(19) Europäisches Patentamt
European Patent Office
Office européen des brevets

(11) EP 4 674 436 A1

(12) **EUROPEAN PATENT APPLICATION**
published in accordance with Art. 153(4) EPC

(43) Date of publication:
**07.01.2026 Bulletin 2026/02**

(21) Application number: **24763134.4**

(22) Date of filing: **27.02.2024**

(51) International Patent Classification (IPC):
***A61K 47/68*** (2017.01) ***A61K 39/395*** (2006.01)
***C07K 16/30*** (2006.01) ***A61P 35/00*** (2006.01)
***C07K 16/28*** (2006.01) ***C12N 15/13*** (2006.01)
***A61K 31/704*** (2006.01)

(52) Cooperative Patent Classification (CPC):
**A61K 31/704; A61K 39/395; A61K 47/68; A61P 35/00; C07K 16/00; C07K 16/28; C07K 16/30**

(86) International application number:
**PCT/CN2024/078768**

(87) International publication number:
**WO 2024/179453 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.02.2023 CN 202310170388**

(71) Applicant: **CSPC Megalith Biopharmaceutical Co., Ltd.**
**Shijiazhuang, Hebei 050025 (CN)**

(72) Inventors:
- **XIE, Ru**
  **Shijiazhuang, Hebei 050025 (CN)**
- **HAN, Weiqiang**
  **Shijiazhuang, Hebei 050025 (CN)**
- **WANG, Xuming**
  **Shijiazhuang, Hebei 050025 (CN)**
- **SU, Li**
  **Shijiazhuang, Hebei 050025 (CN)**
- **CAO, Weirong**
  **Shijiazhuang, Hebei 050025 (CN)**
- **ZHANG, Di**
  **Shijiazhuang, Hebei 050025 (CN)**
- **YUAN, Can**
  **Shijiazhuang, Hebei 050025 (CN)**
- **TIAN, Fangyuan**
  **Shijiazhuang, Hebei 050025 (CN)**
- **HUI, Xiwu**
  **Shijiazhuang, Hebei 050025 (CN)**
- **YAO, Bing**
  **Shijiazhuang, Hebei 050025 (CN)**
- **WANG, Chao**
  **Shijiazhuang, Hebei 050025 (CN)**

(74) Representative: **Gille Hrabal Partnerschaftsgesellschaft mbB**
**Patentanwälte**
**Brucknerstraße 20**
**40593 Düsseldorf (DE)**

Remarks:
The complete document including Reference Table(s) and the Sequence Listing(s) can be downloaded from the EPO website

(54) **PHARMACEUTICAL COMPOSITION OF RECOMBINANT HUMANIZED ANTI-NECTIN-4 MONOCLONAL ANTIBODY-MMAE CONJUGATE DRUG**

(57) A pharmaceutical composition, comprising (i) an antibody-drug conjugate, (ii) a histidine buffer, (iii) trehalose, and (iv) a surfactant; and a method for preparing the pharmaceutical composition.

EP 4 674 436 A1

## Description

### TECHNICAL FIELD

The present disclosure relates to a specific antibody-drug conjugate formulation and a preparation method for the formulation.

### BACKGROUND

Nectin-4, also known as poliovirus-like receptor 4 (PVRL4), is a type I transmembrane protein encoded by the *Nectin-4* gene of the Nectin family. It is mainly expressed in the placenta and is weakly to moderately expressed in normal human tissues including the skin, bladder, salivary glands, esophagus, breast, and stomach. However, it is overexpressed in tissues of various cancers (breast cancer, lung cancer, ovarian cancer, etc.) and found to be expressed in 62% of triple-negative breast cancer. Overexpression of Nectin-4 in cancer progression can promote angiogenesis within the tumor and promote tumor growth. In addition, the PI3K/AKT signaling pathway is involved in the promotion of Nectin-4-mediated cancer cell proliferation. The overexpression of Nectin-4 molecules is associated with poor prognosis of lung cancer, breast cancer, and ovarian cancer, and the high expression of Nectin-4 is a risk factor for lymph node metastasis in patients with papillary thyroid carcinoma (PTC). Nectin-4 depletion can effectively inhibit the proliferation and invasion of two PTC cell lines (i.e., TPC1 and KTC-1) *in vitro* and induce apoptosis. The overexpression of Nectin-4 in human esophageal cancer tissues is closely associated with the size of tumors, the depth of tumor infiltration, and the poor prognosis of patients. The intervention on the expression of Nectin-4 in esophageal cancer cell lines shows that the increased expression of Nectin-4 can significantly promote cell viability, migration and invasion, and tumorigenesis. The difference in expression levels between tumor tissues and normal tissues makes Nectin-4 a highly favored target in the field of ADC drug development. In studies of antibody and antibody-drug conjugate (ADC) formulations, the formation of aggregates and the generation of degradation products cause pharmaceutically undesirable side effects, which pose an increased risk of immunogenicity or venous disorders associated with patients receiving drug therapy. For the reasons described above, when formulating related formulations, it is necessary to inhibit the formation of aggregates and the generation of degradation products, and in view of this, researchers have studied various formulation forms and formulation formulas (e.g., aqueous injection and lyophilized injection forms) of pharmaceutical compositions. Similarly, the study on pharmaceutical formulations of antibody-drug conjugates faces more technical challenges, as it is necessary to consider not only the specific properties of the antibody moiety but also those of the drug-linker moiety. For example, antibody-drug conjugates may experience shedding of small-molecule toxins during storage, which influences the efficacy and toxicity of the related drugs.

Therefore, there is still a need in the art for a stable antibody-drug conjugate formulation, particularly a formulation suitable for the antibody-drug conjugate of the present disclosure.

### SUMMARY

The present disclosure relates to a recombinant humanized anti-Nectin-4 monoclonal antibody-MMAE drug conjugate for injection. The antibody is a self-developed recombinant humanized anti-Nectin-4 monoclonal antibody, which is obtained by site-specific modification of glutamine on the heavy chain of an antibody by means of microbial transglutaminase (mTgase) catalysis, followed by conjugation with a small-molecule microtubule inhibitor drug LND1002. LND1002 is a compound in which an amino-PEG-Val-Cit linker and MMAE (monomethyl auristatin E) are bridged together via p-amino-benzyloxycarbonyl (pABC). Val-Cit is a dipeptide that is sensitive to proteases in lysosomes. Upon internalization of the ADC, Val-Cit is cleaved, triggering the self-degradation of the linker and the self-clearance of pABC. This releases MMAE as a free toxin without the linker, which inhibits tubulin polymerization in the cell division cycle, leading to G2/M phase arrest and tumor cell apoptosis, and finally leading to tumor cell death and exhibiting an anti-tumor effect. The average drug-to-antibody ratio (DAR) is 2.0, with over 70% of the product in the DAR2 form, indicating high product homogeneity. This is beneficial to the quality control of the drug and the maintenance of good batch-to-batch consistency. In addition, the inventors of the present disclosure optimize parameters such as pH value, buffer salt, stabilizer and surfactant through extensive experiments to determine the optimal formula composition of the enzymatic site-specific antibody-drug conjugate, and prepare a lyophilized powder of the Nectin-4 antibody-drug conjugate by lyophilization to ensure the stability of the physicochemical properties of the antibody moiety while minimizing the shedding of free drugs, so that the lyophilized powder can be stored at 2-8 °C for a long time. The present disclosure specifically relates to the following aspects:

In one aspect, the present disclosure relates to a pharmaceutical composition comprising (i) an antibody-drug conjugate, (ii) a buffer, (iii) a stabilizer, and (iv) a surfactant.

In some embodiments, provided is the antibody-drug conjugate, which is formed by conjugating a drug-linker

represented by the following formula (formula I) to an anti-Nectin-4 monoclonal antibody:

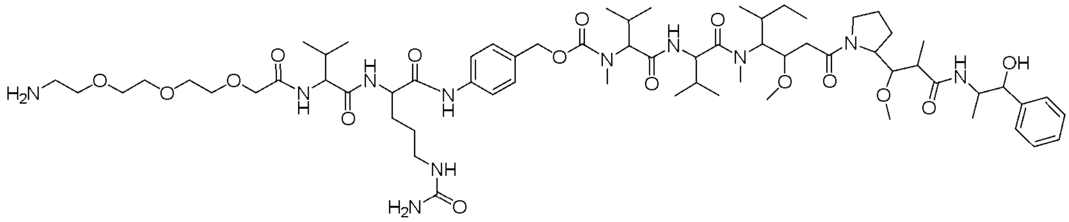

formula I.

In some embodiments, the buffer includes, but is not limited to: acetate, succinate, gluconate, histidinate, oxalate, lactate, phosphate, citrate, tartrate, fumarate, glycylglycine, and other organic acid buffers.

In some embodiments, the histidinate buffer is a buffer comprising histidinate ions. Examples of histidinate buffers include histidine-histidine hydrochloride, histidine-acetate, histidine-phosphate, histidine-sulfate, etc., wherein the histidine-acetate buffer is formulated with histidine and acetic acid, and the histidine-histidine hydrochloride buffer is formulated with histidine and histidine hydrochloride. Similarly, the succinate buffer may be formulated with succinic acid-sodium succinate, and the citrate buffer may be formulated with citric acid-sodium citrate.

In some embodiments, the histidine-histidine hydrochloride buffer is preferred.

In some embodiments, the stabilizer includes, but is not limited to: saccharides (such as sucrose and trehalose), polyols (such as mannitol and sorbitol), and amino acids (L-serine, sodium glutamate, alanine, glycine, sarcosine, etc.).

In some embodiments, the surfactant includes, but is not limited to: polysorbate 20 or polysorbate 80.

In some embodiments, the pharmaceutical composition has a pH of 5.0-7.4, preferably 5.0-6.5, and most preferably about 6.0.

In some embodiments, the pharmaceutical composition of the present disclosure further comprises water.

In some embodiments, the components of the pharmaceutical composition of the present disclosure include: histidine-histidine hydrochloride: 5-30 mM, for example, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, or about 30 mM; polysorbate 20 or polysorbate 80: 0.02-0.08 wt%, for example, about 0.02 wt%, about 0.03 wt%, about 0.04 wt%, about 0.05 wt%, about 0.06 wt%, about 0.07 wt%, or about 0.08 wt%; trehalose: 4-8 wt%, for example, about 4 wt%, about 5 wt%, about 5.5 wt%, about 6 wt%, about 6.5 wt%, about 7 wt%, about 7.5 wt%, or about 8 wt%; and the antibody-drug conjugate: 5-50 mg/mL, for example, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, or about 50 mg/mL; pH 5.0-6.5, for example, about 5.0, about 5.5, about 6.0, or about 6.5.

In some embodiments, the components of the pharmaceutical formulation of the present disclosure include: histidine-histidine hydrochloride: 5-30 mM, polysorbate 20 or polysorbate 80: 0.02-0.08 wt%, trehalose: 5-7 wt%, and the antibody-drug conjugate: 5-50 mg/mL, pH 5.0-6.5.

In some embodiments, the components of the pharmaceutical formulation of the present disclosure include: histidine-histidine hydrochloride: 10-30 mM, polysorbate 20 or polysorbate 80: 0.02-0.04 wt%, trehalose: 5-6 wt%, and the antibody-drug conjugate: 10-20 mg/mL, pH 5.0-6.0.

In some preferred embodiments, the components of the pharmaceutical formulation of the present disclosure include: histidine-histidine hydrochloride: 20 mM, polysorbate 20: 0.02 wt%, trehalose: 5.5 wt%, and the antibody-drug conjugate: 10 mg/mL, pH 6.0.

In some preferred embodiments, the pharmaceutical formulation of the present disclosure comprises: the antibody-drug conjugate: 10 mg/mL, histidine: 1.4 mg/mL, histidine hydrochloride: 2.3 mg/mL, polysorbate 20: 0.2 mg/mL, and trehalose: 55 mg/mL, pH 6.0.

In some embodiments, the pharmaceutical composition of the present disclosure further comprises water.

In some preferred embodiments, the components of the pharmaceutical formulation of the present disclosure consist of histidine-histidine hydrochloride: 20 mM, polysorbate 20: 0.02 wt%, trehalose: 5.5 wt%, the antibody-drug conjugate: 10 mg/mL, and water for injection, pH 6.0.

In some preferred embodiments, the components of the pharmaceutical formulation of the present disclosure consist of histidine-histidine hydrochloride: 20 mM, polysorbate 20: 0.02 wt%, trehalose: 5.5 wt%, and the antibody-drug conjugate: 10 mg/mL, pH 6.0.

In some preferred embodiments, the liquid pharmaceutical composition may be prepared into a lyophilized formulation, a liquid formulation, or a powder for injection.

In another aspect, the present disclosure further relates to a method for producing the pharmaceutical

composition, which comprises the steps of preparing an anti-Nectin-4 antibody-drug conjugate, buffer-exchanging the antibody-drug conjugate into other components of a liquid pharmaceutical composition, performing sterilizing filtration, and performing aseptic filling.

In some embodiments, the present disclosure further relates to a method for producing the pharmaceutical composition, which comprises the following steps:

(1) preparing an aqueous solution comprising a predetermined amount of the following substances: (i) an antibody-drug conjugate, (ii) a buffer, (iii) a stabilizer, and (iv) a surfactant;
(2) if necessary, adjusting the pH of the aqueous solution to a predetermined value; (3) performing sterilizing filtration and aliquoting the solution into an injection vial.

In some preferred embodiments, the present disclosure further provides a method for preparing the pharmaceutical composition, which comprises:

(1) buffer exchange for an antibody-drug conjugate by ultrafiltration: A buffer (e.g., a histidine-histidine hydrochloride solution) at the same concentration as a formula amount of the final product is prepared, and buffer exchange is performed by using a 30 kD ultrafiltration membrane cartridge to exchange the original affinity chromatography solution into the buffer (e.g., the histidine-histidine hydrochloride solution), wherein the buffer exchange by ultrafiltration is performed at a factor of no less than 100, the pH of the exchange solution is controlled to be consistent with that of the final product, and the concentration of the antibody-drug conjugate is not less than that of the final product (optimally at a factor of $\geq$ 1.2);
(2) preparation of a semi-finished product dilution: A formulation amount of the final product is calculated according to the formula amount of the product and the concentration of the antibody-drug conjugate solution after buffer exchange by ultrafiltration, and the required amounts of auxiliary materials are calculated, so as to formulate a semi-finished product dilution as follows: About 80% water for injection is added into a formulation vessel, a buffer salt, a surfactant and a stabilizer (e.g., histidine, histidine hydrochloride, polysorbate 20 and trehalose) are sequentially weighed and added into the preparation vessel, and the mixture is stirred for mixing; after the auxiliary materials are completely dissolved, water for injection is added to bring the volume to a formulation amount, and the mixture is stirred for uniform mixing, and then filtered for later use;
(3) preparation of the finished product: The prepared semi-finished product dilution is added to the formulation solution containing the antibody-drug conjugate drug solution after buffer exchange by ultrafiltration, to bring the volume to a formulation amount of the semi-finished product with the dilution, the mixture is stirred for uniform mixing, and sampled for detection of protein content, pH value and microbial limit, and after sterilizing filtration, the solution is aliquoted into sterilized injection vials according to product specifications, and the vials are stoppered and capped to obtain the finished product.

In another aspect, the present disclosure further provides a lyophilized formulation prepared by lyophilizing the liquid pharmaceutical composition; in some embodiments, a preparation method for the lyophilized formulation comprises the steps of pre-freezing, vacuumizing, primary drying, and secondary drying.

In some embodiments, the secondary drying is performed at a temperature of about 30 °C.

In some embodiments, the primary drying process is performed under a condition of about -25 °C, 0.15 mbar.

In another aspect, the present disclosure provides use of the pharmaceutical composition described above in the preparation of a medicament for treating a tumor (including cancer).

In another aspect, the present disclosure provides a method for treating a tumor (including cancer), which comprises administering to a subject in need the pharmaceutical composition described above.

In another aspect, the present disclosure provides the pharmaceutical composition described above for use in treating a tumor (including cancer).

In some embodiments, the cancer described above is Nectin-4 positive cancer.

In some embodiments, the tumor (including cancer) described above includes, but is not limited to, a hematological tumor or a solid tumor.

In some embodiments, the tumor (including cancer) described above is a hematological tumor, preferably lymphoma or leukemia, including but not limited to: myeloma, B-cell lymphoma, mantle cell lymphoma, non-Hodgkin's B-cell lymphoma, non-Hodgkin's T-cell lymphoma, cutaneous lymphoma, anaplastic large cell lymphoma, multiple myeloma, indolent non-Hodgkin's lymphoma, plasmacytoma, chronic lymphocytic leukemia, small lymphocytic lymphoma, or follicular lymphoma. In some embodiments, the hematological tumor is recurrent or refractory.

In some embodiments, the tumor (including cancer) described above is a solid tumor, including but not limited to: respiratory system tumors, gastrointestinal tumors, urinary system tumors, male reproductive organ tumors, female reproductive organ tumors, skin cancer, endothelial tumors, brain tumors, nervous system tumors, and endocrine organ

tumors.

In some embodiments, the respiratory system tumors include, but are not limited to: lung cancer, nasopharyngeal cancer, and laryngeal cancer.

In some embodiments, the gastrointestinal tumors include, but are not limited to: esophageal cancer, gastric cancer, colorectal cancer, liver cancer, pancreatic cancer, and cholangiocarcinoma.

In some embodiments, the urinary system tumors include, but are not limited to: kidney cancer, renal pelvis and ureteral cancer, bladder cancer, and urethral cancer.

In some embodiments, the male reproductive organ tumors include, but are not limited to, penile cancer, prostate cancer, or testicular cancer.

In some embodiments, the female reproductive organ tumors include, but are not limited to, breast cancer, vulval cancer, vaginal cancer, cervical cancer, endometrial cancer, or ovarian cancer.

In some embodiments, the nervous system tumors include, but are not limited to: astrocytoma, oligodendroglioma, ependymoma, medulloblastoma, and meningioma.

In some embodiments, the brain tumors include, but are not limited to: glioma, neurocytoma, embryonal mesenchymal tumors, stromal tumors, epithelial tumors, teratoma, and pinealoma. In some embodiments, the skin cancer includes, but is not limited to: cutaneous melanoma or non-melanoma skin cancer.

In some embodiments, the tumor is a solid tumor, including but not limited to: bladder cancer, brain cancer, breast cancer, cervical cancer, thoracic tumors, endometrial cancer, esophageal squamous carcinoma, gastric cancer, head tumors, pancreatic cancer, cholangiocarcinoma, colorectal cancer, eye cancer, head and neck squamous cell carcinoma, urothelial carcinoma, kidney cancer, liver cancer, lymph node cancer, lung cancer, oral cancer, neck tumors, ovarian cancer, prostate cancer, testicular cancer, laryngeal cancer, uterine cancer, melanoma, salivary gland cancer, fibrosarcoma, soft tissue sarcoma, and osteosarcoma. In some embodiments, the cancer described above is recurrent or refractory.

In some embodiments, the breast cancer includes: ductal carcinoma, lobular carcinoma, medullary carcinoma, colloid carcinoma, tubular carcinoma, inflammatory breast cancer, and triple-negative breast cancer (TNBC).

In some embodiments, the ovarian cancer includes: epithelial ovarian tumors, such as adenocarcinoma in the ovary and adenocarcinoma with metastasis from the ovary to the abdominal cavity.

In some embodiments, the leukemia includes: acute myeloid leukemia (AML), acute lymphoblastic leukemia, hairy cell leukemia, myelodysplasia, myeloproliferative disorders, NK cell leukemia (e.g., blastic plasmacytoid dendritic cell neoplasm), , chronic myeloid leukemia (CML), mastocytosis, chronic lymphocytic leukemia (CLL), multiple myeloma (MM), and myelodysplastic syndrome (MDS).

In some embodiments, the pancreatic cancer is acinar cell adenocarcinoma of the pancreas, ductal cell adenocarcinoma of the pancreas, or stage IV pancreatic cancer.

In some embodiments, the lung cancer includes non-small cell lung cancer (NSCLC) and small cell lung cancer (SCLC); in some embodiments, the non-small cell lung cancer (NSCLC) includes, but is not limited to: squamous cell carcinoma, adenocarcinoma, and large cell carcinoma; in some embodiments, the lung cancer is recurrent; in some embodiments, the lung cancer is recurrent squamous cell lung cancer or (advanced) stage IV squamous cell lung cancer. In some embodiments, the prostate cancer is metastatic castration-resistant prostate cancer (mCRPC).

In some embodiments, the pharmaceutical composition of the present disclosure can be selectively used as a medicament for drug therapy, which is a primary method for treating cancer, and thus can delay the development of cancer cells, inhibit the growth of cancer cells, and further kill cancer cells. These effects may free cancer patients from symptoms caused by cancer, or achieve improvement in the QOL (quality of life score) of cancer patients, thereby obtaining therapeutic effects by maintaining the lives of cancer patients. Even if the pharmaceutical compositions and treatment methods of the present disclosure do not achieve the killing of cancer cells, they can achieve higher QOL (quality of life score) of cancer patients by inhibiting or controlling the growth of cancer cells, while achieving longer survival.

In another aspect, the pharmaceutical compositions of the present disclosure may be used alone, and in addition, they may be used in combination with other therapies, and may be combined with surgery, radiotherapy, hormone therapy, etc.

In some embodiments, the pharmaceutical compositions of the present disclosure may be administered in combination with an additional anti-cancer therapeutic agent. Therefore, the anti-cancer effect can be enhanced. Examples of additional cancer therapeutic agents for this purpose include 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastine, vinorelbine, everolimus, tanespimycin, bevacizumab, oxaliplatin, and lapatinib, but the examples are not limited thereto, as long as they are agents having anti-tumor activity.

In some embodiments, the antibody comprises a heavy chain and a light chain, wherein the heavy chain comprises three CDR regions having amino acid sequences set forth in SEQ ID NOs: 1, 2 and 3, respectively, and/or the light chain comprises three CDR regions having amino acid sequences set forth in SEQ ID NOs: 4, 5 and 6, respectively.

In some embodiments, the heavy chain comprises a heavy chain variable region having an amino acid sequence

set forth in SEQ ID NO: 7, and/or the light chain comprises a light chain variable region selected from an amino acid sequence set forth in any one of SEQ ID NOs: 8-10.

In some embodiments, the heavy chain of the antibody comprises a heavy chain constant region having an amino acid sequence set forth in SEQ ID NO: 11 or 12, and/or the light chain of the antibody comprises a light chain constant region selected from an amino acid sequence set forth in SEQ ID NO: 13.

In some embodiments, the antibody is a monoclonal antibody or a bispecific antibody, preferably a monoclonal antibody, more preferably a humanized antibody, and most preferably a fully human antibody.

In some specific embodiments, the antibody or the functional fragment thereof of the present disclosure has ADCC activity.

In some specific embodiments, the antibody or the functional fragment thereof of the present disclosure has CDC activity.

In some specific embodiments, the antibody or the functional fragment thereof of the present disclosure is an IgG1κ antibody.

The antibody-drug conjugate (ADC) described herein refers to a conjugate having a cytotoxic drug conjugated to an antibody, wherein an antigen for the antibody is expressed on the surface of cancer cells and the antibody further binds to an antigen capable of cell internalization, thereby enabling selective delivery of the drug to cancer cells, which causes accumulation of the drug within the cancer cells and kills the cancer cells.

The histidine hydrochloride described herein is also referred to as L-histidine monohydrochloride monohydrate, and similarly, the histidine-histidine hydrochloride described herein is also referred to as L-histidine, L-histidine monohydrochloride monohydrate.

The numbering scheme for CDRs of the antibody of the present disclosure is IMGT.

## BRIEF DESCRIPTION OF THE DRAWING

FIG. 1: schematic diagram of mTgase enzymatic ADC conjugation reaction.
FIG. 2: schematic structural diagram of ADC, in which
LND1002 represents the linker + drug moiety. The LND1002 on the right side shows the complete structure, and the circled part indicates the amide bond linkage between the linker in LND1002 and the antibody. The ADCs of the examples of the present application are site-specific antibody-drug conjugates. Each molecule comprises one anti-Nectin-4 monoclonal antibody conjugated to one molecule of MMAE at the amino acid at position Q294 (i.e., Kabat numbering Q295) of each heavy chain via a linker ($NH_2$-$PEG_3$-Val-Cit-pABC). The antibody and the linker are linked by a stable amide bond (isopeptide bond), and the average drug-to-antibody ratio (DAR) is 2.0.
FIG. 3: experiment on the endocytosis effect of SWY2001-Ab1-LND1002 in SK-BR-3 cells.
FIG. 4: experiment on the endocytosis effect of SWY2001-Ab1-LND1002 in T47D cells.
FIG. 5: experiment on the *in vivo* inhibitory effect of SWY2001-Ab1-LND1002 on the PC3-Nectin-4 stably transfected cell line-derived tumor in mice. Note: mpk = mg/kg.
FIG. 6: experiment on the *in vivo* inhibitory effect of SWY2001-Ab1-LND1002 on HT-1376 tumor in mice.

## Examples:

### Example 1: Preparation and Efficacy Evaluation of Nectin-4 Antibody-Drug Conjugate

#### 1.1 Preparation of antibody-drug conjugate

LND1002 (dissolved in DMSO at a ratio of 1 g:5 mL, having a structure shown in FIG. 2), a 10× reaction buffer, an anti-Nectin-4 antibody (obtained by expressing a vector containing sequences related to heavy chain and light chain variable regions in a host cell by using genetic engineering techniques, followed by purification; for the related sequences, refer to the sequences of the humanized antibody SWY2001-Ab1 in Table 30, with constant regions HC1 + LC), mTgase (transglutaminase, used for catalyzing transglutamination at the receptor glutamine near the N-glycosylation site, which can specifically catalyze transglutamination at Kabat numbering Q295, with the sequence set forth in SEQ ID NO: 14 (see Table 30)), and 20% $H_2O$ were sequentially added to an EP tube. After sealing and vortexing, the mixture was incubated at 30°C for a reaction for no more than 72 h. When the conjugation ratio reached 95%, the reaction was stopped, followed by immediate purification of the reaction mixture to obtain the anti-Nectin-4 antibody-drug conjugate SWY2001-Ab1-LND1002. The reaction conditions were as follows:

Table 1: Conjugation reaction system

| | |
|---|---|
| Batch | Mab-MMAE |
| Reaction scale | 10 mg |
| Antibody concentration | 10 mg/mL |
| Molar ratio of drug to antibody | 30:1 |
| mTgase | 0.6 mg/mL |
| Buffer | 50 mM Tris-OAc + 2 mM EDTA + 0.02% PS20 pH 8.0 |
| Reaction condition | 30 °C 72 h |
| DAR | 2.039 |

According to the experimental results, it was confirmed that the transglutaminase catalytic site of the antibody of the present application is located at position Q295 near the N-glycosylation site.

As determined by the experiment, the modification ratio of the antibody-drug conjugate after 72 h of reaction was 96.32%, the DAR value was about 2, and the main physicochemical properties met the requirements for further experiments.

1.2 Endocytosis effect of antibody-drug conjugate

SK-BR-3 cells and T47D cells (human breast cancer cells) were collected and resuspended in a culture medium. The target cells were gently pipetted several times to form a single-cell suspension, and the cell viability and cell count were determined by trypan blue staining. The cell density was adjusted to $1 \times 10^5$ cells/mL. The cells were seeded in a confocal 96-well plate cell culture dish at 100 $\mu$L/well, with $1 \times 10^4$ cells per well. The Zenon™ pHrodo™ iFL-labeled ADC was added to the 96-well plate at a final concentration of 2 $\mu$g/mL, and then the plate was placed in an incubator at 37 °C with 5% $CO_2$ for continuous culture for 24 h. All images were observed and captured using a 20× objective lens of a laser confocal microscope.

According to the experimental results shown in FIGs. 3 and 4, SWY2001-Ab1-LND1002 was endocytosed in cells and located in an acidic lysosome environment, with an endocytosis rate significantly higher than that of the control drug PADCEV. For antibody-drug conjugates, a high endocytosis rate generally indicates strong ability to enter tumor cells, which enables more efficient toxin release and faster onset of drug action. Therefore, the ability of the antibody-drug conjugate obtained in the present application to enter tumor cells is significantly superior to that of the control drug PADCEV.

*1.3 In vitro* pharmacodynamic experiment on ADC

The target cells were collected and resuspended into a single-cell suspension, and the cell viability and cell count were determined by trypan blue staining. The cell density was adjusted to $1 \times 10^5$ cells/mL. The cell suspension was added to a 96-well black flat-bottom cell culture plate at 100 $\mu$L/well. The diluted test sample was added to the 96-well black flat-bottom cell culture plate (pre-seeded with cells) at 20 $\mu$L/well. The plate was incubated in a cell incubator (37 °C, 5% $CO_2$) for 66 $\pm$ 3 h. A resazurin sodium solution (0.03%) was added at 20 $\mu$L/well. The plate was incubated at 37 °C for 3-4 h. Fluorescence values were read on a microplate reader at 550 nm/610 nm. The data was plotted using Prism or similar software, and the half-maximal inhibitory concentration IC50 values of the reference standard and the sample were calculated by fitting. The output parameter C was IC50 in ng/mL.

According to the experimental results shown in Table 2, SWY2001-Ab1-LND1002 could inhibit the growth of 293T-Nectin-4, SK-BR-3, and PC3-Nectin-4 cancer cells *in vitro.* The *in vitro* killing data of the stably transfected 293T-Nectin-4 cell line showed that the killing activity of SWY2001-Ab1-LND1002 was slightly superior to that of PADECV; the *in vitro* killing data of SK-BR-3 showed that the killing activity of SWY2001-Ab1-LND1002 was 4 times that of PADCEV; the *in vitro* killing data of PC3-Nectin-4 showed that no significant differences in the killing activity were observed between SWY2001-Ab1-LND1002 and PADCEV.

Table 2: *In vitro* inhibitory effect of SWY2001-Ab1-LND1002 on several cancer cells

| Cell | SWY2001-Ab1-LND1002 (IC50 ng/mL) | PADCEV (IC50 ng/mL) |
|---|---|---|
| 293T-Nectin-4 | 2.6789 | 3.8262 |
| SK-BR-3 | 334.62 | 1487.85 |

(continued)

| Cell | SWY2001-Ab1-LND1002 (IC50 ng/mL) | PADCEV (IC50 ng/mL) |
|---|---|---|
| PC3-Nectin-4 | 40.14 | 34.15 |

1.4. *In vivo* pharmacodynamic experiment on ADC

1. *In vivo* pharmacodynamic experiment on the inhibition of prostate cancer

In this experiment, age-appropriate female NUNU mice were selected to be inoculated with PC3-Nectin-4 stably transfected cells (nude mouse xenograft tumor model of human prostate cancer, highly expressing Nectin-4). When the tumor volume reached about 190 $mm^3$ (d19 after inoculation), 42 animals with good tumor growth were selected. The animals were evenly divided into 6 groups by tumor volume (D0): a solvent control group, a Nectin-4-mab (SWY2001-Ab1 monoclonal antibody) 1 mg/kg group, a PADCEV 1 mg/kg group, and SWY2001-Ab1-LND1002 0.5 mg/kg, 1 mg/kg and 2 mg/kg groups, with 7 animals in each group. After administration, the body weight of the mice was measured and the data were recorded. The tumor growth was dynamically observed by measuring the tumor diameters at different time points after administration. At the end of the experiment on d17 (17 days after administration), the mice were euthanized by carbon dioxide asphyxiation, and then the tumors were excised and weighed.

Under the experimental conditions (see FIG. 5), SWY2001-Ab1-LND1002 could inhibit the tumor growth in a dose-dependent manner ($P < 0.05$). At the dose of 1 mg/kg, the tumor inhibitory rate of SWY2001-Ab1-LND1002 was significantly better than that of PADCEV (58.3% vs 39.0%). The tumor weight inhibitory rates of the Nectin-4-mab (Nectin-4 monoclonal antibody) 1 mg/kg group, the PADCEV 1 mg/kg group, and the SWY2001-Ab1-LND1002 0.5 mg/kg, 1 mg/kg and 2 mg/kg groups were 28.2%, 39.0%, 22.1%, 58.3%, and 79.4%, respectively.

2. *In vivo* pharmacodynamic experiment on the inhibition of bladder cancer

In this experiment, age-appropriate female NUNU mice were inoculated with HT-1376 cells at $5 \times 10^6$ cells/mouse. When the tumor volume reached about 100 $mm^3$ (d21 after inoculation), 12 animals with good tumor growth were selected. The animals were evenly divided into 3 groups by tumor volume (D0): A: a solvent control group, 4 animals, 0.9% sodium chloride injection (0.9% INJ NS (normal saline), solvent control group); B: a PADCEV experimental group, 4 animals, 3 mg/kg PADCEV (single dose); and C: an SWY2001-Ab1-LND1002 experimental group, four animals, 3 mg/kg SWY2001-Ab1-LND1002 (single dose). After administration, the body weight of the mice was measured and the data were recorded. The tumor growth was dynamically observed by measuring the tumor diameters at different time points after administration. At the end of the experiment on d22, the mice were euthanized by carbon dioxide asphyxiation, and then the tumors were excised and weighed.

According to the experimental results shown in FIG. 6, under the experimental conditions, the tumor inhibitory rates of the PADCEV 3 mg/kg group and the SWY2001-Ab1-LND1002 3 mg/kg group were 42.1% and 49.4%, respectively. Compared with the solvent control group, the SWY2001-Ab1-LND1002 3 mg/kg (single dose) group could significantly inhibit the tumor growth. Compared with the positive control PADCEV 3 mg/kg (single dose) group, the SWY2001-Ab1-LND1002 3 mg/kg group had a significantly improved inhibitory effect on tumors.

1.5. Experimental results of ADC stability

Plasma stability experiment

In this experiment, the stability of SWY2001-Ab1-LND1002 and MMAE in the plasma of SD rats, cynomolgus monkeys, and humans were determined by *in vitro* plasma incubation at 37 °C. In addition, PBST was selected as a negative control group to investigate the reliability of the entire experimental system. Incubation samples were collected from each incubation group at 0 h, 24 h, 48 h (D2), 72 h (D3), 96 h (D4), 168 h (D7), and 336 h (D14). The concentrations of MMAE in the plasma of various species and PBST were determined by LC-MS/MS. The mean percentage of MMAE generated after incubation of SWY2001-Ab1-LND1002 in plasma of various species and PBST are shown in Table 3.

Table 3: Average percentage (%) of MMAE generation in plasma of various species

| Incubate | Species | Incubation concentration μM | Average percentage of MMAE generation (%) | | | | | | |
|---|---|---|---|---|---|---|---|---|---|
| | | | Incubation time (h) | | | | | | |
| | | | 0 | 24 | 48 | 72 | 96 | 168 | 336 |
| ADC | Human | 0.02 | 0.00 | 0.00 | 0.10 | 0.13 | 0.21 | 0.47 | 0.82 |
| | | 0.2 | 0.00 | 0.05 | 0.10 | 0.16 | 0.22 | 0.43 | 0.93 |
| | | 2 | 0.00 | 0.05 | 0.10 | 0.17 | 0.22 | 0.48 | 1.00 |
| | Monkey | 0.02 | 0.00 | 0.00 | 0.11 | 0.19 | 0.24 | 0.44 | 0.72 |
| | | 0.2 | 0.00 | 0.06 | 0.11 | 0.19 | 0.26 | 0.47 | 0.72 |
| | | 2 | 0.00 | 0.05 | 0.12 | 0.19 | 0.26 | 0.52 | 0.75 |
| | Rat | 0.02 | 0.00 | 0.06 | 0.14 | 0.22 | 0.29 | 0.45 | 0.97 |
| | | 0.2 | 0.00 | 0.07 | 0.15 | 0.24 | 0.30 | 0.55 | 1.20 |
| | | 2 | 0.00 | 0.07 | 0.14 | 0.23 | 0.40 | 0.74 | 1.04 |
| | PBST | 0.02 | 0.00 | 0.00 | 0.00 | 0.14 | 0.10 | 0.18 | 0.20 |
| | | 0.2 | 0.00 | 0.03 | 0.05 | 0.08 | 0.10 | 0.18 | 0.24 |
| | | 2 | 0.00 | 0.02 | 0.05 | 0.08 | 0.11 | 0.18 | 0.25 |

The experimental results showed that within the experimental concentration range, after incubation of antibody-drug conjugates at three concentrations in the plasma of humans, monkeys and rats for 336 h, the generation amount of small-molecule MMAE accounted for about 1% of the theoretical amount, demonstrating that the antibody-drug conjugates have good stability.

## Example 2: Screening Experiment on Optimal pH Value

The pH value is an important factor influencing the stability of biological product formulations, which has a regulatory effect on the charge distribution on the protein surface, affects the intermolecular and intramolecular forces of the protein, and affects the conformational stability, colloidal stability, and chemical stability of the protein. Samples with different pH values were prepared at an ADC concentration of 10 mg/mL using a citric acid-disodium hydrogen phosphate buffer system, with a pH range of 4.5-8.0. The samples with different pH values were aliquoted into injection vials and subjected to stability studies at 2-8 °C and high temperature (50 °C) to evaluate the thermal stability, the stability of molecular size variants (SEC-HPLC), and the stability of charge variants (CEX-HPLC).

Detection methods:

DSC: A test sample and a buffer that is the same as that used for sample preparation were diluted to a protein concentration of 1 mg/mL. 350 μL of the test sample and the corresponding buffer were each added to corresponding positions in a 96-well plate, and the 96-well plate was placed in a sample chamber with the temperature set at 15 °C. Instrument parameter settings: initial temperature: 20 °C, final temperature: 100 °C, and heating rate: 90 °C/min.

SEC-HPLC: Analysis and detection were performed using a chromatographic column packed with a chromatographic gel suitable for separating proteins with molecular weights of 10-500 kD according to General Chapter 0512 "High-Performance Liquid Chromatography" of the Chinese Pharmacopoeia (2020 Edition, Volume IV). The proportions of the protein monomer peak, high-molecular-weight species (HMWS) peak, and low-molecular-weight species (LMWS) peak were calculated by the area normalization method.

CEX-HPLC: Analysis and detection were performed using a cation exchange column as a chromatographic column according to General Chapter 0512 "High-Performance Liquid Chromatography" of the Chinese Pharmacopoeia, (2020 Edition, Volume IV). The proportions of the acidic peak, main peak, and basic peak of the test sample were calculated by the area normalization method.

Detection results:

Table 4: DSC detection results

| pH value | $T_{onset}$(°C) | $T_{m1}$(°C) | $T_{m2}$(°C) |
|---|---|---|---|
| 4.5 | 48.82 | 55.09 | 76.97 |
| 5.0 | 54.29 | 59.26 | 78.77 |
| 5.5 | 58.41 | 64.16 | 79.07 |
| 6.0 | 60.76 | 69.72 | 77.86 |
| 6.5 | 61.01 | 70.21 | 76.54 |
| 7.0 | 60.66 | 70.17 | 75.22 |
| 7.5 | 59.24 | 70.10 | 73.60 |
| 8.0 | 58.87 | 67.46 | 72.46 |

Table 5: SEC-HPLC detection results

| pH value | 4 °C, 72 h | | | 50 °C, 72 h | | |
|---|---|---|---|---|---|---|
| | Polymer (%) | Monomer (%) | Fragment (%) | Polymer (%) | Monomer (%) | Fragment (%) |
| 5.0 | 1.03 | 96.56 | 2.41 | 46.77 | 51.24 | 2.00 |
| 5.5 | 1.13 | 96.74 | 2.14 | 6.55 | 91.03 | 2.43 |
| 6.0 | 1.17 | 96.89 | 1.95 | 4.86 | 92.77 | 2.38 |
| 6.5 | 1.21 | 96.83 | 1.97 | 8.37 | 89.30 | 2.34 |
| 7.0 | 1.28 | 96.90 | 1.84 | 6.16 | 91.40 | 2.44 |
| 7.5 | 1.35 | 96.89 | 1.77 | 6.57 | 90.58 | 2.85 |

Table 6: CEX-HPLC detection results

| pH value | 4 °C, 72 h | | | 50 °C, 72 h | | |
|---|---|---|---|---|---|---|
| | Acidic peak (%) | Main peak (%) | Basic peak (%) | Acidic peak (%) | Main peak (%) | Basic peak (%) |
| 5.0 | 16.40 | 64.35 | 19.26 | 23.99 | 38.03 | 37.98 |
| 5.5 | 16.18 | 65.16 | 18.66 | 26.42 | 48.12 | 25.46 |
| 6.0 | 16.53 | 65.16 | 18.32 | 26.00 | 46.32 | 27.68 |
| 6.5 | 16.56 | 65.33 | 18.12 | 29.50 | 43.66 | 26.84 |
| 7.0 | 16.73 | 65.32 | 17.98 | 37.46 | 39.74 | 22.80 |
| 7.5 | 17.07 | 65.39 | 17.55 | 55.82 | 29.71 | 14.47 |

Based on the comprehensive analysis of the thermal stability results of the product at different pH values and the degradation trends of the molecular size variants and charge variants of the product after forced high-temperature treatment, pH 5.5-6.5 was preferred.

**Example 3: Screening Experiment on Buffer Components**

Buffer salts have important influences on maintaining the pH value, physicochemical properties and stability of formulations. Screening of buffer salts and evaluation of their effects were conducted within the preferred pH range. Buffer salts including citric acid-sodium citrate, histidine-histidine hydrochloride, and the like were used to prepare different buffer salt samples with an ADC concentration of 10 mg/mL. The samples were aliquoted into injection vials and subjected to stability studies at high temperature (40 °C) to evaluate the thermal stability, the stability of molecular size variants and charge variants, and the changes in the content of free toxin LND1002. The detection methods for the thermal stability and the stability of the molecular size variants and charge variants were the same as those in Example 2.

Detection methods:

Free LND1002 content: The test sample was precipitated with acetonitrile, followed by an ice bath. The supernatant was collected and subjected to analysis and detection using a chromatographic column packed with an octadecyl-bonded silica gel according to General Chapter 0512 "High-Performance Liquid Chromatography" of the Chinese Pharmacopoeia (2020 Edition, Volume IV). The content of free LND1002 in the test sample was calculated by the external standard method.

Detection results:

Table 7: DSC detection results

| Buffer system | pH value | $T_{onset}$(°C) | $T_{m1}$(°C) | $T_{m2}$(°C) |
|---|---|---|---|---|
| Histidine-histidine hydrochloride | 5.5 | 54.95 | 63.23 | 78.47 |
| | 6.0 | 60.36 | 65.94 | 79.53 |
| | 6.5 | 63.68 | 68.49 | 79.01 |
| Citric acid-sodium citrate | 5.5 | 54.95 | 64.57 | 78.31 |
| | 6.0 | 59.85 | 66.64 | 77.35 |
| | 6.5 | 59.85 | 67.97 | 75.91 |

Table 8: SEC-HPLC detection results (40 °C, 2 weeks)

| Buffer system | pH value | Main peak (%) | | |
|---|---|---|---|---|
| | | 0 d | 40 °C 7 d | 40 °C 14 d |
| Histidine-histidine hydrochloride | 5.5 | 97.43 | 97.36 | 97.07 |
| | 6.0 | 97.39 | 97.02 | 96.54 |
| | 6.5 | 97.53 | 96.24 | 94.76 |
| Citric acid-sodium citrate | 5.5 | 97.30 | 96.81 | 96.21 |
| | 6.0 | 97.06 | 96.62 | 96.15 |
| | 6.5 | 97.13 | 96.33 | 95.43 |

Table 9: CEX-HPLC detection results (40 °C, 2 weeks)

| Buffer system | pH value | Main peak (%) | | |
|---|---|---|---|---|
| | | 0 d | 40 °C 7 d | 40 °C 14 d |
| Histidine-histidine hydrochloride | 5.5 | 61.87 | 52.16 | 50.13 |
| | 6.0 | 61.56 | 51.27 | 47.19 |
| | 6.5 | 61.33 | 49.42 | 43.92 |
| Citric acid-sodium citrate | 5.5 | 61.22 | 49.71 | 43.86 |
| | 6.0 | 60.73 | 49.83 | 44.27 |
| | 6.5 | 60.44 | 49.12 | 43.50 |

Table 10: Detection results of free LND1002 (40 °C, 2 weeks)

| Buffer system | Free LND1002 content (%) | | | |
|---|---|---|---|---|
| | pH value | 0 d | 40 °C 7 d | 40 °C 14 d |
| Histidine-histidine hydrochloride | 5.5 | 0.00027 | 0.00257 | 0.00268 |
| | 6.0 | 0.00059 | 0.00170 | 0.00255 |
| | 6.5 | 0.000 17 | 0.00146 | 0.00237 |
| Citric acid-sodium citrate | 5.5 | 0.00015 | 0.00260 | 0.00358 |
| | 6.0 | 0.00015 | 0.00245 | 0.00313 |
| | 6.5 | 0.00016 | 0.00199 | 0.00276 |

According to the above experimental results, it could be seen that the thermal stability of the histidine buffer system was superior to that of the citrate buffer system; the stability of molecular size variants and charge variants at a lower pH value was superior to that at a higher pH value, but the content of free toxin LND1002 gradually decreased as the pH value increased. Based on the comprehensive analysis, pH 6.0, histidine buffer system was preferred.

## Example 4: Screening Experiment on Stabilizer

The addition of saccharides (such as sucrose and trehalose) at a certain ratio can not only reduce the aggregates of protein drugs, achieving the purpose of stabilizing monoclonal antibody drugs, but also adjust the osmotic pressure of the product and serve as an excipient for the lyophilized product. Sucrose and trehalose were separately added to the preferred pH 6.0, histidine-histidine hydrochloride buffer salt to prepare samples with an ADC concentration of 10 mg/mL. The samples were aliquoted into injection vials and subjected to stability studies at high temperature (40 °C) to evaluate the thermal stability, physical stability, and chemical stability. Meanwhile, lyophilization pre-experiments were performed on the samples with different stabilizers to evaluate the differences in the appearance and water content between the two lyophilized products. The detection methods were the same as those in Example 2.

Detection results:

Table 11. DSC detection results

| Stabilizer | $T_{onset}$(°C) | $T_{m1}$ (°C) | $T_{m2}$(°C) |
|---|---|---|---|
| Trehalose | 59.53 | 66.51 | 80.08 |
| Sucrose | 60.08 | 66.78 | 80.12 |

Table 12: SEC-HPLC detection results (40 °C, 2 weeks)

| Stabilizer | Main peak (%) | | |
|---|---|---|---|
| | 0 d | 40 °C 7 d | 40 °C 14 d |
| Trehalose | 96.97 | 96.42 | 95.13 |
| Sucrose | 97.49 | 96.59 | 95.30 |

Table 13: CEX-HPLC detection results (40 °C, 2 weeks)

| Stabilizer | Main peak (%) | | |
|---|---|---|---|
| | 0 d | 40 °C 7d | 40 °C 14 d |
| Trehalose | 61.23 | 51.09 | 43.49 |
| Sucrose | 61.41 | 49.67 | 42.43 |

Table 14: Detection results of free LND1002 (40 °C, 2 weeks)

| Stabilizer | Free LND1002 content (%) | | |
|---|---|---|---|
| | 40 °C 0 d | 40 °C 7 d | 40 °C 14 d |
| Trehalose | 0.00083 | 0.00277 | 0.00253 |
| Sucrose | 0.00068 | 0.00241 | 0.00254 |

Table 15: Detection results of basic evaluation indicators for lyophilized powder

| Stabilizer | Appearance of lyophilized powder | Water content (%) | Reconstitution time |
|---|---|---|---|
| Trehalose | White porous solid with slight shrinkage at the bottom | 1.26 | ≤1min |
| Sucrose | White porous solid with slightly more pronounced shrinkage at the bottom | 1.16 | ≤1min |

According to the above experimental results, it could be seen that the thermal stability of the product was slightly increased after the addition of the stabilizer; no significant differences were observed between two saccharides in terms of the molecular size variants, charge variants and the free LND1002 content of the samples; no significant differences were observed between two saccharides as lyophilization excipients in terms of the water content of the lyophilized powder, and the two lyophilized powders had good reconstitution properties. The morphology of the bottom of the lyophilized powder was observed, and it was found that the shrinkage of the bottom of the sucrose lyophilized powder was slightly more pronounced than that of trehalose. In conclusion, trehalose is preferably used as the stabilizer.

## Example 5: Screening Experiment on Surfactant

The biomacromolecular protein drugs are prone to aggregation and adsorption during storage. The addition of surfactants can reduce the aggregation of the proteins during stirring, shaking, freeze-thawing, and lyophilization, preventing the proteins from being adsorbed on the surface of the container. Based on the preferred pH, buffer, and stabilizer, different concentrations of surfactants (including polysorbate 20) were added to prepare samples with an ADC concentration of 10 mg/mL. The samples were aliquoted into injection vials and subjected to stability studies under shaking and freeze-thawing conditions. Changes in sub-visible particles in the samples under different conditions were detected using a Micro-Flow Imaging System (Flowcam).

Table 16: Influence of shaking on insoluble particles of samples with the different addition amounts of polysorbate 20

| PS20 content / Particle | 0 | | 0.005% | | 0.02% | | 0.04% | |
|---|---|---|---|---|---|---|---|---|
| | T0 | Shaking | T0 | Shaking | T0 | Shaking | T0 | Shaking |
| Total number of particles (P/mL) | 6432 | 1389017 | 510 | 1737 | 510 | 1232 | 372 | 844 |
| NSP 2~10 μm (P/mL) | 1011 | 324584 | 208 | 487 | 86 | 228 | 71 | 124 |
| NSP 10+μm (P/mL) | 43 | 18484 | 8 | 23 | 8 | 10 | 25 | 30 |

Table 17: Influence of freeze-thawing on insoluble particles of samples with different addition amounts of polysorbate 20

| PS20 content / Particle | 0 | | 0.005% | | 0.02% | | 0.04% | |
|---|---|---|---|---|---|---|---|---|
| | T0 | Freeze-thawing | T0 | Freeze-thawing | T0 | Freeze-thawing | T0 | Freeze-thawing |
| Total number of particles (P/mL) | 6432 | 52694 | 510 | 7087 | 510 | 10703 | 372 | 11527 |
| NSP 2~10 μm (P/mL) | 1011 | 9681 | 208 | 878 | 86 | 1344 | 71 | 1395 |
| NSP 10+μm (P/mL) | 43 | 357 | 8 | 15 | 8 | 30 | 25 | 28 |

The results showed that the addition of the surfactant polysorbate 20 could effectively control the increase in the number of particles, and the addition of 0.005% polysorbate 20 could significantly inhibit the formation of particles. Based on the comprehensive analysis of experimental data described above, 0.02% polysorbate 20 was preferably used as a preferred ingredient of the ADC formulation to prevent the formation of aggregates in the ADC during long-term storage.

## Example 6: Lyophilization Process Development Experiment

Lyophilization process development was performed using a preferred formula composition: 10 mg/mL ADC, 20 mmol/L histidine-histidine hydrochloride, 0.02% polysorbate 20, and 5.5% trehalose, pH 6.0 as an example of the basic formula. A sample with the formula composition was detected for glass transition temperature and collapse temperature. Low-temperature DSC detection showed that the glass transition temperature of the sample was -29.08 °C, and lyophilization microscopy showed that the collapse temperature of the sample was -26.0 °C. Pre-freezing: The pre-freezing temperature was set at -40 °C according to the glass transition temperature. Firstly, the platen temperature was rapidly reduced to -10 °C and maintained for 30 min. Then, the products were each rapidly cooled (≥ 2 °C/min) or slowly cooled (≤ 0.5 °C/min) to a pre-freezing temperature of -40 °C, and maintained at this temperature for no less than 180 min. The lyophilization process was performed under the same primary drying and secondary drying conditions. During the lyophilization process, the samples were placed at different positions along the diagonal of the platen (positions A and E were mainly proximal to the door or side wall, where thermal radiation was more significant, leading to relatively low water content; the center point C was the most proximal to the inside, where sublimation was mainly achieved by heat conduction, leading to a relatively high water content. Among them, position A was located at the connection between the front door and side wall, experiencing the most severe external thermal radiation, while positions B and D were more proximal to the inside than positions A and E). No significant differences in the water content of the lyophilized powders were observed between the two pre-freezing methods. Small ice crystals were observed to form during rapid freezing, but no increase in the water content was observed when compared with slow freezing. Therefore, the rapid freezing method was preferably selected for pre-freezing.

Table 18: Influence of rapid freezing and slow freezing at different positions on water content

| Sample position No. | Water content (%) | |
|---|---|---|
| | Slow freezing | Rapid freezing |
| A | 1.39 | 1.16 |
| B | 1.34 | 1.30 |
| C | 1.64 | 1.46 |
| D | 1.35 | 1.64 |
| E | 1.46 | 1.39 |
| Average value | 1.44 | 1.39 |

Primary drying: The primary drying process removes more than 90% of the water in the product through sublimation. This part of water is mainly free water. During primary drying, the product temperature decreased due to the endothermic nature of sublimation. The sample temperature remained lower than the platen temperature throughout the

process. Once sublimation was completed and most of the water was removed, the product temperature increased to the platen temperature. Thus, the primary drying was completed. In order to increase the sublimation rate of the product, the platen temperature should be set above the collapse temperature allowed for the product, while ensuring that the temperature of the unsublimed part does not exceed the collapse temperature thereof. The rapid freezing process was preferably selected for pre-freezing. The influence of primary drying temperatures (-25 °C and -20 °C) and front chamber pressures (0.15 mbar and 0.2 mbar) was investigated by designing different experimental combinations. The lyophilization process was performed under the same pre-freezing and secondary drying conditions, and three primary drying processes were performed. The results showed that the lower the set value of the primary drying platen temperature and the lower the set value of the front chamber pressure, the longer the time for primary drying. Compared with 0.2 mbar, reducing the set value of the front chamber pressure to 0.15 mbar improved the shrinkage at the bottom layer of the lyophilized powder. The appearance of the product after primary drying at - 25 °C was better than that at -20 °C. Based on the comprehensive analysis of the above factors, -25 °C, 0.15 mbar was preferably selected as the conditions for the primary drying process.

Table 19: Influence of primary drying processes at different positions on water content

| Sample position No. | Water content (%) | | |
|---|---|---|---|
| | -20 °C 0.2 mbar | -20 °C 0.15 mbar | -25 °C 0.15 mbar |
| A | 1.16 | 1.03 | 1.12 |
| B | 1.30 | 1.16 | 1.25 |
| C | 1.46 | 1.21 | 1.33 |
| D | 1.64 | 1.17 | 1.27 |
| E | 1.39 | 1.28 | 1.35 |
| Average value | 1.39 | 1.17 | 1.26 |

Secondary drying: after the primary drying is completed, about 10% of water in the product is adsorbed on the capillary tube walls and polar groups of the dried substance. When the bound water reaches a certain content, it provides conditions for the growth and reproduction of microorganisms and the occurrence of certain chemical reactions. This part of water is the bound water adsorbed on the drug by weak intermolecular forces such as van der Waals forces and hydrogen bonds. To remove this part of water, additional energy is required to overcome the intermolecular forces. Therefore, the platen temperature should be set below the maximum temperature allowed for the product. For biological products, the secondary drying temperature should not be too high. Therefore, 30 °C is preferably selected as the secondary drying temperature. Since the main factor influencing the water content of the lyophilized powder during the secondary drying is the set platen temperature, the front chamber pressure is set at 0.15 mbar, which is consistent with that in primary drying.

The key quality attributes of the lyophilized samples of the preferred process before and after lyophilization were compared. The results (Table 20) showed that the quality of the lyophilized products was not significantly changed before and after lyophilization, indicating that the lyophilization process does not influence the product quality.

Table 20: Influence of lyophilization process on product quality

| Investigation item | Dosage form | |
|---|---|---|
| | Bulk solution before lyophilization | Lyophilized powder |
| Water content | - | 0.67% |
| Reconstitution time | - | 1min |
| SEC-HPLC | HMWS:2.0% | HMWS:1.8% |
| | Monomer: 96.8% | Monomer: 97.0% |
| | LMWS:1.2% | LMWS:1.2% |
| NR-CE-SDS | Monomer: 96.1% | Monomer: 97.1% |
| | LMWS:3.9% | LMWS:2.9% |

(continued)

| Investigation item | Dosage form | |
|---|---|---|
| | Bulk solution before lyophilization | Lyophilized powder |
| CEX-HPLC | Acidic peak: 16.4% | Acidic peak: 16.4% |
| | Main peak: 59.6% | Main peak: 59.4% |
| | Basic peak: 24.0% | Basic peak: 24.3% |
| DAR distribution | DAR value: 2.0 | DAR value: 2.0 |
| | DAR2:81.8% | DAR2:81.8% |
| Unconjugated antibody content | 0.36% | 0.34% |
| Free LND1002 content | 0.00207% | 0.00137% |
| Free MMAE content | 0.000087% | 0.000124% |
| Antigen binding activity | 100% | 98% |
| Biological activity | 100% | 102% |

**Example 7: Formulation Stability**

Three batches of samples were prepared under GMP conditions with optimized formula composition (the same as that in Example 6) and lyophilization process. Stability studies were performed on the three batches of samples, with all detection methods for research indicators validated for methodology to ensure accuracy and reliability. Stability studies at 5 ± 3 °C were performed on the bulk solution before lyophilization and the finished product after lyophilization.

Detection methods:

SEC-HPLC: Detection was performed using a chromatographic column packed with a chromatographic gel suitable for separating proteins with molecular weights of 10-500 kD (such as XBridge® BEH200Å, 7.8 × 300 mm, 200 Å, 3.5 μm, or other suitable chromatographic columns) according to General Chapter 0512 "High-Performance Liquid Chromatography" of the Chinese Pharmacopoeia (2020 Edition, Volume IV), with chromatographic conditions as follows: mobile phase: 100 mmol/L phosphate buffer, 100 mmol/L NaCl, 10% isopropanol, pH 6.7 + 0.1; flow rate: 0.8 mL/min; and detection wavelength: 280 nm. An appropriate amount of the test sample solution was injected into a liquid chromatograph, and the proportions of the protein monomer peak, high-molecular-weight species (HMWS) peak, and low-molecular-weight species (LMWS) peak were calculated by the area normalization method.

CEX-HPLC: Detection was performed using an ion exchange column (such as Thermo ProPac WCX-10, 4 × 250 mm, or other suitable chromatographic columns) as a chromatographic column according to General Chapter 0512 "High-Performance Liquid Chromatography" of the Chinese Pharmacopoeia (2020 Edition, Volume IV), with chromatographic conditions as follows: mobile phase A: 10 mmol/L PB, pH 6.5, and mobile phase B: 10 mmol/L PB, 300 mmol/L NaCl, pH 6.5; flow rate: 0.8 mL/min; and detection wavelength: 280 nm. An appropriate amount of the test sample solution was injected into a liquid chromatograph for gradient elution, and the proportions of the acidic peak, main peak, and basic peak of the test sample were calculated by the area normalization method.

R-CE-SDS: Detection was performed according to General Chapter 3127 "Determination of Size Variants of Monoclonal Antibodies" of the Chinese Pharmacopoeia (2020 Edition, Volume IV).

The proportions of the heavy chain, light chain, and non-glycosylated heavy chain peaks were calculated by the area normalization method.

NR-CE-SDS: Detection was performed according to General Chapter 3127 "Determination of Size Variants of Monoclonal Antibodies" of the Chinese Pharmacopoeia (2020 Edition, Volume IV). The proportions of the monomer peak and low-molecular-weight species (LMWS) peak were calculated by the area normalization method.

icIEF: Detection was performed according to General Chapter 3129 "Monoclonal Antibody Charge Variant Determination Method" of the Chinese Pharmacopoeia (2020 Edition, Volume IV). According to isoelectric point (PI) characteristics of different charge variants, the charge variants were separated by capillary electrophoresis (General Chapter 0542). The isoelectric points of the charge variants were determined and the relative percentage contents were calculated.

DAR distribution: Detection was performed using a chromatographic column packed with a tetraalkyl-bonded silica gel (such as PLRP-S 1000A, 2.1 × 50 mm, particle size: 5 μm, or other suitable chromatographic columns) according

to General Chapter 0512 "High-Performance Liquid Chromatography" of the Chinese Pharmacopoeia (2020 Edition, Volume IV), with chromatographic conditions as follows: mobile phase A: 0.1% (v/v) TFA aqueous solution, and mobile phase B: 0.1% (v/v) TFA in acetonitrile solution; column temperature: 80 °C; flow rate: 0.25 mL/min; and detection wavelength: 280 nm. An appropriate amount of the test sample solution was injected into a liquid chromatograph for gradient elution, and the DAR value and the DAR2 content were calculated.

Amount of unconjugated antibody: Detection was performed using the same detection method as for DAR distribution, and the content of the DAR0 peak was reported as the amount of unconjugated antibody.

Free MMAE content: Detection was performed using a chromatographic column packed with an octadecyl-bound silica gel (such as Waters ACQUITY UPLC® BEH-C18, 2.1 × 50 mm, particle size: 1.7 μm, or other suitable chromatographic columns) according to General Chapter 0512 "High-Performance Liquid Chromatography" and General Chapter 0431 "Mass Spectrometry" of the Chinese Pharmacopoeia (2020 Edition, Volume IV), with chromatographic conditions as follows: mobile phase A: an aqueous solution containing 0.1% TFA, and mobile phase B: an acetonitrile solution containing 0.1% TFA; column temperature: 40 °C; flow rate: 0.4 mL/min; and detection wavelength: 250 nm. 50 μL (10 mg/mL) of the test sample was taken, an internal standard working solution was added, and the mixture was precipitated with acetonitrile at a ratio of 1:3. The mixture was placed in an ice bath at -20 °C for 60 min, and then centrifuged. The supernatant was collected and loaded, and different concentrations of MMAE were loaded as controls. After gradient elution by liquid chromatography, the sample entered the mass spectrometer detector. Parameters such as the capillary and cone voltage of the mass spectrometer, ion source temperature, collision energy, and atomization flow rate were set for analysis. The free MMAE content in the test sample was calculated according to the standard curve.

Free MMAE content (%) = MMAE detection concentration/ADC concentration × 100% Antigen binding activity: Determination was performed by the enzyme-linked immunosorbent assay (ELISA) method.

Biological activity: Determination was performed by the cell proliferation inhibition assay method.

The study results showed that the quality of the product after lyophilization was more stable than that in the liquid state; the low-molecular-weight fragments of the product in the liquid state significantly increased compared with that in the lyophilized state; when the product was in the liquid state, charge variants were more likely to form basic variants, and free MMAE was more likely to be produced, with a shedding rate more significant than that in the lyophilized powder. Therefore, the lyophilized dosage form was more favorable for long-term storage of the product.

Table 21: Stability results of key quality indicators for GMP batch 1

| Investigation item | GMP batch 1 | | | |
|---|---|---|---|---|
| | Bulk solution (5 ± 3 °C) | | Lyophilized powder (5 ± 3 °C) | |
| | 0 months | 6 months | 0 months | 6 months |
| SEC-HPLC | HMWS:0.9% | 0.7% | HMWS:0.9% | 0.7% |
| | Monomer: 97.5% | 96.9% | Monomer: 97.6% | 97.9% |
| | LMWS:1.6% | 2.4% | LMWS:1.5% | 1.4% |
| NR-CE-SDS | Monomer: 96.1% | 95.2% | Monomer: 96.3% | 96.5% |
| | LMWS:3.2% | 4.3% | LMWS:3.1% | 2.8% |
| R-CE-SDS | HC+LC:97.5% | 97.1% | HC+LC:97.3% | 97.1% |
| | NG-HC:1.1% | 1.3% | NG-HC:0.8% | 1.5% |
| CEX-HPLC | Acidic peak: 22.7% | 22.4% | Acidic peak: 22.4% | 22.4% |
| | Main peak: 58.1% | 52.4% | Main peak: 57.3% | 54.2% |
| | Basic peak: 19.2% | 25.2% | Basic peak: 20.4% | 23.4% |
| icIEF | Acidic peak: 40.5% | 41.6% | Acidic peak: 40.6% | 39.9% |
| | Main peak: 57.5% | 56.3% | Main peak: 57.4% | 57.9% |
| | Basic peak: 2.0% | 2.1% | Basic peak: 2.0% | 2.1% |
| DAR | DAR value: 2.0 | 2.0 | DAR value: 2.0 | 2.0 |
| | DAR2:82.1% | 82.2% | DAR2:81.9% | 82.3% |
| Amount of unconjugated antibody | 0.34% | 0.29% | 0.42% | 0.30% |
| Amount of free MMAE | 0.000012% | 0.00039% | 0.000044% | 0.00014% |

(continued)

| Investigation item | GMP batch 1 | | | |
|---|---|---|---|---|
| | Bulk solution (5 ± 3 °C) | | Lyophilized powder (5 ± 3 °C) | |
| | 0 months | 6 months | 0 months | 6 months |
| Antigen binding activity | 114% | 108% | 99% | 103% |
| Biological activity | 119% | 105% | 95% | 117% |

Table 22: Stability results of key quality indicators for GMP batch 2

| Investigation item | GMP batch 2 | | | |
|---|---|---|---|---|
| | Bulk solution (5 ± 3 °C) | | Lyophilized powder (5 ± 3 °C) | |
| | 0 months | 6 months | 0 months | 6 months |
| SEC-HPLC | HMWS:0.6% | 0.5% | HMWS:0.5% | 0.5% |
| | Monomer: 97.9% | 97.1% | Monomer: 98.5% | 98.0% |
| | LMWS:1.5% | 2.4% | LMWS: 1.0% | 1.4% |
| NR-CE-SDS | Monomer: 97.3% | 95.7% | Monomer: 97.2% | 97.0% |
| | LMWS:2.2% | 3.8% | LMWS:2.3% | 2.5% |
| R-CE-SDS | HC+LC:97.7% | 97.8% | HC+LC:97.8% | 97.9% |
| | NG-HC:0.9% | 0.7% | NG-HC:0.8% | 0.9% |
| CEX-HPLC | Acidic peak: 19.9% | 20.7% | Acidic peak: 19.8% | 20.4% |
| | Main peak: 57.8% | 54.1% | Main peak: 58.1% | 56.3% |
| | Basic peak: 22.3% | 25.3% | Basic peak: 22.1% | 23.3% |
| icIEF | Acidic peak: 37.4% | 40.2% | Acidic peak: 37.0% | 36.8% |
| | Main peak: 60.8% | 57.9% | Main peak: 60.1% | 59.7% |
| | Basic peak: 1.9% | 2.0% | Basic peak: 2.9% | 1.9% |
| DAR | DAR value: 2.0 | 2.0 | DAR value: 2.0 | 2.0 |
| | DAR2:82.5% | 82.6% | DAR2:83.0% | 82.8% |
| Amount of unconjugated antibody | 0.34% | 0.29% | 0.31% | 0.30% |
| Amount of free MMAE | 0.000012% | 0.00046% | 0.000055% | 0.00017% |
| Antigen binding activity | 98% | 95% | 107% | 82% |
| Biological activity | 113% | 109% | 107% | 104% |

Table 23: Stability results of key quality indicators for GMP batch 3

| Investigation item | GMP batch 3 | | | |
|---|---|---|---|---|
| | Bulk solution (5 ± 3 °C) | | Lyophilized powder (5 ± 3 °C) | |
| | 0 months | 6 months | 0 months | 6 months |
| SEC-HPLC | HMWS:0.7% | 0.5% | HMWS:0.7% | 0.5% |
| | Main peak: 97.8% | 97.2% | Monomer: 97.8% | 98.1% |
| | LMWS:1.6% | 2.3% | LMWS:1.6% | 1.4% |
| NR-CE-SDS | Monomer: 97.2% | 96.0% | Monomer: 97.2% | 97.0% |
| | LMWS:2.2% | 3.5% | LMWS:2.2% | 2.7% |
| R-CE-SDS | HC+LC:98.1% | 97.8% | HC+LC:98.2% | 97.9% |
| | NG-HC:0.6% | 0.7% | NG-HC:0.7% | 0.8% |

(continued)

| Investigation item | GMP batch 3 | | | |
|---|---|---|---|---|
| | Bulk solution (5 ± 3 °C) | | Lyophilized powder (5 ± 3 °C) | |
| | 0 months | 6 months | 0 months | 6 months |
| CEX-HPLC | Acidic peak: 19.9% | 20.1% | Acidic peak: 19.8% | 19.9% |
| | Main peak: 57.4% | 54.6% | Main peak: 57.1% | 56.6% |
| | Basic peak: 22.8% | 25.3% | Basic peak: 23.1% | 23.5% |
| icIEF | Acidic peak: 38.2% | 40.1% | Acidic peak: 37.1% | 39.6% |
| | Main peak: 59.4% | 58.0% | Main peak: 60.6% | 58.0% |
| | Basic peak: 2.5% | 1.8% | Basic peak: 2.3% | 2.3% |
| DAR | DAR value: 2.0 | 2.0 | DAR value: 2.0 | 2.0 |
| | DAR2:82.4% | 82.3% | DAR2:82.7% | 82.3% |
| Amount of unconjugated antibody | 0.37% | 0.35% | 0.40% | 0.36% |
| Amount of free MMAE | 0.000027% | 0.00042% | 0.000044% | 0.00014% |
| Antigen binding activity | 120% | 106% | 108% | 87% |
| Biological activity | 117% | 116% | 119% | 100% |

Based on the above experimental results, it could be seen that the main physicochemical properties of the ADC lyophilized powders of different batches were kept stable, maintaining good stability and batch-to-batch consistency.

**Example 8: Stability of Liquid Formulation at 40 °C for 14 Days**

Preparation of Ab1-ADC, Ab2-ADC, and Ab3-ADC molecules: An Ab1 antibody molecule (see Table 30; variable region sequence: SWY2001-Ab1, and constant region: HC1 + LC), an Ab2 antibody molecule (see Table 30; variable region sequence: SWY2001-Ab2, and constant region: HC1 + LC), and an Ab3 antibody molecule (see Table 30; variable region sequence: SWY2001-Ab3, and constant region: HC1 + LC) were each mixed with LND1002, mTgase, and a reaction buffer. The mixtures were uniformly mixed and then placedat 30 °C for a reaction for no more than 72 h. (Specifically, the preparation can be performed with reference to 1.1 of Example 1). When the conjugation ration reached 95%, the reaction was stopped, followed by immediate purification of the reaction mixtures to obtain the Ab1-ADC, Ab2-ADC, and Ab3-ADC molecules with a DAR value of about 2.0, respectively.

Preparation of a reference formulation molecule: A molecule corresponding to the marketed drug PADCEV (same target and drug) was prepared. The antibody was first constructed, and a corresponding antibody molecule was obtained by culture and purification. The antibody molecule was buffer-exchanged into a corresponding buffer, and a TCEP reducing agent was added. The mixture was heated in a water bath at 37 °C for 1 h, and then a corresponding amount of payload (MC-VC-pABC-MMAE) was added. The mixture was reacted at room temperature for 30 min, and then a corresponding amount of L-cysteine was added. After 20 min, the reaction was stopped, followed by immediate purification of the reaction mixture to obtain a reference molecule with a DAR value of about 4.0.

Stability comparison of Ab1-ADC, Ab2-ADC, Ab3-ADC, and the reference molecule: Four ADC molecules were each buffer-exchanged into water without any buffer system. The buffer-exchanged samples were filtered through a 0.22 μm filter membrane. After filtration, the samples were aliquoted into 2.0 mL cleaned and sterilized injection vials at 1.0 mL/vial. The stability of the four molecules was analyzed at a high temperature of 40 °C, and the evaluation indicators included appearance, molecular size variants (SEC-HPLC), charge variants (CEX-HPLC), and free MMAE content (RP-HPLC/MS).

Analysis methods:

Molecular size variant (SEC-HPLC): Detection was performed using a chromatographic column packed with a chromatographic gel suitable for separating proteins with molecular weights of 10-500 kD (such as XBridge® BEH200Å, 7.8 × 300 mm, 200 Å, 3.5 μm, or other suitable chromatographic columns), with chromatographic conditions as follows: mobile phase: 100 mmol/L phosphate buffer, 100 mmol/L NaCl, 10% isopropanol, pH 6.7 ± 0.1; flow rate: 0.8 mL/min; and detection wavelength: 280 nm. An appropriate amount of the test sample solution was injected into a liquid chromatograph,

and the proportions of the protein monomer peak, high-molecular-weight species (HMWS) peak, and low-molecular-weight species (LMWS) peak were calculated by the area normalization method.

Charge variants (CEX-HPLC): Detection was performed using an ion exchange column (such as Thermo ProPac WCX-10, 4 × 250 mm, or other suitable chromatographic columns) as a chromatographic column, with chromatographic conditions as follows: mobile phase A: 10 mmol/L PB, pH 6.5, and mobile phase B: 10 mmol/L PB, 300 mmol/L NaCl, pH 6.5; flow rate: 0.8 mL/min; and detection wavelength: 280 nm. An appropriate amount of the test sample solution was injected into a liquid chromatograph for gradient elution, and the proportions of the acidic peak, main peak, and basic peak of the test sample were calculated by the area normalization method.

Free MMAE content (RP-HPLC/MS): Detection was performed using a reversed phase chromatographic column (such as Waters ACQUITY UPLC® BEH-C18, 2.1 × 50 mm, particle size: 1.7 μm, or other suitable chromatographic columns) by reverse-phase liquid chromatography-mass spectrometry, with chromatographic conditions as follows: mobile phase A: an aqueous solution containing 0.1% TFA, and mobile phase B: an acetonitrile solution containing 0.1% TFA; column temperature: 40 °C; flow rate: 0.4 mL/min; and detection wavelength: 250 nm. 50 μL (10 mg/mL) of the test sample was taken, an internal standard working solution was added, and the mixture was precipitated with acetonitrile at a ratio of 1:3. The mixture was placed in an ice bath at -20 °C for 60 min, and then centrifuged. The supernatant was collected and loaded, and different concentrations of MMAE were loaded as controls. After gradient elution by liquid chromatography, the sample entered the mass spectrometer detector. Parameters such as the capillary tube and cone voltage of the mass spectrometer, ion source temperature, collision energy, and atomization flow rate were set for analysis. The free MMAE content in the test sample was calculated according to the standard curve.

$$\text{Free MMAE content (\%)} = \text{MMAE detection concentration/ADC concentration} \times 100\%$$

**Table 24: Comparison of high-temperature (40 °C) stability of four molecules in an aqueous solution system**

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab1-ADC | 0 d | Colorless and clear solution | 1.2 | 97.4 | 1.4 | 21.7 | 58.0 | 20.3 | 0.000068 |
| | 40 °C 7 d | Milky white solution | 3.1 | 94.3 | 2.6 | 25.1 | 50.1 | 24.8 | 0.017 |
| | 40 °C 14 d | Milky white solution | 4.8 | 91.5 | 3.7 | 28.9 | 41.9 | 29.2 | 0.035 |
| Ab2-ADC | 0 d | Colorless and clear solution | 1.4 | 97.2 | 1.4 | 21.8 | 58.5 | 19.7 | 0.000071 |
| | 40 °C 7 d | Milky white solution | 3.4 | 93.9 | 2.7 | 25.3 | 51.0 | 23.7 | 0.018 |
| | 40 °C 14 d | Milky white solution | 5.1 | 90.8 | 4.1 | 29.2 | 42.3 | 28.5 | 0.037 |
| Ab3-ADC | 0 d | Colorless and clear solution | 1.2 | 97.5 | 1.3 | 21.8 | 58.2 | 20.0 | 0.000059 |
| | 40 °C 7 d | Milky white solution | 3.0 | 94.5 | 2.5 | 25.6 | 50.5 | 23.9 | 0.017 |
| | 40 °C 14 d | Milky white solution | 5.0 | 91.2 | 3.8 | 29.6 | 42.1 | 28.3 | 0.034 |

(continued)

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Reference molecule | 0 d | Colorless and clear solution | 1.9 | 96.2 | 1.9 | 23.2 | 57.8 | 19.0 | 0.000085 |
| | 40 °C 7 d | Milky white solution | 3.8 | 93.3 | 2.9 | 26.9 | 49.9 | 23.2 | 0.019 |
| | 40 °C 14 d | Milky white solution | 5.6 | 90.1 | 4.3 | 30.8 | 42.2 | 27.0 | 0.038 |

After Ab1-ADC, Ab2-ADC, Ab3-ADC, and the reference molecule were separately placed in an aqueous solution system and subjected to high-temperature treatment at 40 °C for 14 days, a change in the appearance of the products from a clear solution to a milky white solution could be clearly observed. The SEC-HPLC results of Ab1-ADC, Ab2-ADC, Ab3-ADC, and the reference molecule showed a decrease in monomer content, mainly manifested as an increase in the HMWS and LMWS contents. The CEX-HPLC results showed a decrease in the main peak content, accompanied by an increase in the corresponding acidic peak and basic peak contents. The free MMAE content showed an increasing trend. The four molecules showed comparable instability in the aqueous solution system. The reference molecule had a higher DAR value than the Ab1-ADC, Ab2-ADC, and Ab3-ADC molecules, but the change in the free MMAE content did not show a significant increase. Under this condition, the reference molecule showed a lower free MMAE dissociation rate.

Although 0.9% normal saline can be used as an administration system, it is unsuitable for use as a drug storage system. Reasons for unsuitability: Storing the drug in 0.9% normal saline without relevant auxiliary material stabilizers may lead to aggregation and particle formation. Additionally, in the 0.9% normal saline solution, electrostatic shielding may increase protein-protein attraction. Meanwhile, 0.9% normal saline is weakly acidic, which can alter the charge state of ADC molecules, resulting in a decrease in the solubility of the ADC molecules and an increase in the aggregation trend.

Neither the aqueous solution system nor 0.9% normal saline is suitable for use as a storage solution for the four molecules described above. Therefore, a formulation investigation should be performed on the four molecules to investigate their stability.

A preferred formula for the Ab1-ADC formulation was as follows: 10 mg/mL ADC, 20 mmol/L histidine-histidine hydrochloride, 0.02% polysorbate 20, and 5.5% trehalose (pH 6.0). The ADC molecules obtained by conjugating the Ab1, Ab2 and Ab3 molecules to LND1002 and the reference molecule were buffer-exchanged by ultrafiltration. The samples after buffer exchange were filtered through a 0.22 μm filter membrane. After filtration, the samples were aliquoted into 2.0 mL cleaned and sterilized injection vials at 1.0 mL/vial. The stability of the four molecules was analyzed at a high temperature of 40 °C, and the evaluation indicators included appearance, molecular size variants (SEC-HPLC), charge variants (CEX-HPLC), and free MMAE content (RP-HPLC/MS). The analysis methods were the same as above.

**Table 25: Comparison of high-temperature (40 °C) stability of four molecules in a formula system**

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab1-ADC | 0 d | Colorless and clear solution | 1.2 | 97.4 | 1.4 | 21.8 | 57.8 | 20.4 | 0.000033 |
| | 40 °C 7 d | Colorless and clear solution | 1.7 | 96.9 | 1.4 | 24.4 | 52.6 | 23.0 | 0.0015 |
| | 40 °C 14 d | Colorless and clear solution | 2.1 | 96.2 | 1.7 | 26.7 | 48.1 | 25.2 | 0.0032 |
| Ab2-ADC | 0 d | Colorless and clear solution | 1.4 | 97.3 | 1.3 | 21.4 | 58.2 | 20.4 | 0.000038 |
| | 40 °C 7 d | Colorless and clear solution | 1.8 | 96.5 | 1.6 | 24.2 | 53.4 | 22.4 | 0.0016 |
| | 40 °C 14 d | Colorless and clear solution | 2.3 | 95.9 | 1.8 | 26.6 | 48.2 | 25.2 | 0.0034 |
| Ab3-ADC | 0 d | Colorless and clear solution | 1.2 | 97.9 | 0.9 | 21.8 | 58.0 | 20.2 | 0.000027 |
| | 40 °C 7 d | Colorless and clear solution | 1.6 | 97.4 | 1.0 | 24.2 | 53.3 | 22.5 | 0.0014 |
| | 40 °C 14 d | Colorless and clear solution | 2.0 | 96.8 | 1.2 | 26.8 | 48.4 | 24.8 | 0.0029 |
| Reference molecule | 0 d | Colorless and clear solution | 1.9 | 96.5 | 1.6 | 23.9 | 57.3 | 18.8 | 0.000048 |
| | 40 °C 7 d | Colorless and clear solution | 3.8 | 93.6 | 2.6 | 27.7 | 50.8 | 21.5 | 0.0036 |
| | 40 °C 14 d | Colorless and clear solution | 5.6 | 91.3 | 3.1 | 31.1 | 43.5 | 25.4 | 0.0074 |

Ab1-ADC, Ab2-ADC, Ab3-ADC, and the reference molecule were separately placed in the preferred formula for the Ab1-ADC formulation and subjected to high-temperature treatment at 40 °C for 14 days. No changes in the appearance of the products were observed. The SEC-HPLC results showed a decrease in the monomer content. The CEX-HPLC results showed a decrease in the main peak content. The free MMAE content showed an increasing trend. In the preferred formula for the formulation, the changes in the SEC-HPLC monomer content, the CEX-HPLC main peak content, and the free MMAE content of the four molecules were all smaller than those in the aqueous solution system, indicating that the formula has a protective effect on the four molecules. Based on the comprehensive analysis of the changes in the molecular size variants, charge variants, and free MMAE content of the four molecules, in the formulation formula system, both the stability and the improvement in stability compared with the aqueous solution system of Ab1-ADC, Ab2-ADC, and

Ab3-ADC were significantly superior to those of the reference molecule, and no significant differences in the stability were observed among Ab1-ADC, Ab2-ADC, and Ab3-ADC. Among them, the SEC-HPLC monomer content of Ab3-ADC was slightly higher than that of Ab1-ADC and Ab2-ADC, indicating slightly better stability. The SEC-HPLC indexes for molecular size variants, the CEX-HPLC indexes for charge variants, and the free MMAE content corresponding to Ab1-ADC, Ab2-ADC, and Ab3-ADC all showed significant differences compared with those corresponding to the reference molecule. Specifically, the decrease in the SEC-HPLC monomer content and the increase in the HMWS and LMWS contents were significantly lower than those of the reference molecule; the decrease in the CEX-HPLC main peak content for charge variants, and the increase in the acidic peak and basic peak contents were significantly lower than those of the reference molecule; the increase in the free MMAE content was significantly lower than that of the reference molecule. Therefore, the stability of Ab1-ADC, Ab2-ADC, and Ab3-ADC in the formulation formula was significantly superior to that of the reference molecule.

**Example 9: Stability of Liquid at 2-8 °C and -20 °C for 6 Months**

A preferred formula for the Ab1-ADC formulation was as follows: 10 mg/mL ADC, 20 mmol/L histidine-histidine hydrochloride, 0.02% polysorbate 20, and 5.5% trehalose (pH 6.0). The ADC molecules obtained by conjugating the Ab1, Ab2 and Ab3 molecules to LND1002 and the reference molecule were buffer-exchanged by ultrafiltration. The samples after buffer exchange were filtered through a 0.22 μm filter membrane. After filtration, the samples were aliquoted into 2.0 mL cleaned and sterilized injection vials at 1.0 mL/vial. The stability of the four molecules was analyzed at 2-8 °C and -20 °C, and the evaluation indicators included appearance, molecular size variants (SEC-HPLC), charge variants (CEX-HPLC), and free MMAE content (RP-HPLC/MS). The analysis methods were the same as those in Example 8.

**Table 26: Comparison of stability of four molecules at 2-8 °C in a preferred formula system**

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab1-ADC | 0 d | Colorless and clear solution | 1.2 | 97.3 | 1.5 | 21.4 | 58.4 | 20.2 | 0.000028 |
| | 2-8 °C, 3 months | Colorless and clear solution | 1.4 | 97.0 | 1.6 | 22.5 | 56.5 | 21.0 | 0.00026 |
| | 2-8 °C, 6 months | Colorless and clear solution | 1.6 | 96.7 | 1.7 | 23.8 | 54.7 | 21.5 | 0.00049 |
| Ab2-ADC | 0 d | Colorless and clear solution | 1.4 | 97.4 | 1.2 | 22.1 | 57.9 | 20.0 | 0.000032 |
| | 2-8 °C, 3 months | Colorless and clear solution | 1.6 | 97.0 | 1.4 | 23.4 | 55.8 | 20.8 | 0.00028 |
| | 2-8 °C, 6 months | Colorless and clear solution | 1.8 | 96.8 | 1.4 | 24.5 | 54.1 | 21.4 | 0.00053 |

(continued)

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab3-ADC | 0 d | Colorless and clear solution | 1.2 | 97.9 | 0.9 | 20.9 | 58.8 | 20.3 | 0.000018 |
| | 2-8 °C, 3 months | Colorless and clear solution | 1.4 | 97.6 | 1.0 | 22.0 | 57.1 | 20.9 | 0.00023 |
| | 2-8 °C, 6 months | Colorless and clear solution | 1.5 | 97.4 | 1.1 | 23.2 | 55.2 | 21.6 | 0.00044 |
| Reference molecule | 0 d | Colorless and clear solution | 1.9 | 96.7 | 1.4 | 22.5 | 57.6 | 19.9 | 0.000039 |
| | 2-8 °C, 3 months | Colorless and clear solution | 2.5 | 95.9 | 1.6 | 24.6 | 54.3 | 21.1 | 0.00058 |
| | 2-8 °C, 6 months | Colorless and clear solution | 2.9 | 95.1 | 2.0 | 27.1 | 50.7 | 22.2 | 0.0013 |

**Table 27: Comparison of stability of four molecules at -20 °C in a preferred formula system**

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab1-ADC | 0 d | Colorless and clear solution | 1.2 | 97.3 | 1.5 | 21.4 | 58.4 | 20.2 | 0.000028 |
| | -20 °C, 3 months | Colorless and clear solution | 1.3 | 97.3 | 1.4 | 21.7 | 58.0 | 20.3 | 0.000041 |
| | -20 °C, 6 months | Colorless and clear solution | 1.3 | 97.2 | 1.5 | 21.9 | 57.7 | 20.4 | 0.000062 |
| Ab2-ADC | 0 d | Colorless and clear solution | 1.4 | 97.4 | 1.2 | 22.1 | 57.9 | 20.0 | 0.000032 |
| | -20 °C, 3 months | Colorless and clear solution | 1.5 | 97.3 | 1.2 | 22.5 | 57.5 | 20.0 | 0.000047 |
| | -20 °C, 6 months | Colorless and clear solution | 1.5 | 97.3 | 1.2 | 22.7 | 57.1 | 20.2 | 0.000069 |

(continued)

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab3-ADC | 0 d | Colorless and clear solution | 1.2 | 97.9 | 0.9 | 20.9 | 58.8 | 20.3 | 0.000018 |
| | -20 °C, 3 months | Colorless and clear solution | 1.3 | 97.8 | 0.9 | 21.2 | 58.4 | 20.4 | 0.000032 |
| | -20 °C, 6 months | Colorless and clear solution | 1.3 | 97.8 | 0.9 | 21.4 | 58.2 | 20.4 | 0.000049 |
| Reference molecule | 0 d | Colorless and clear solution | 1.9 | 96.7 | 1.4 | 22.5 | 57.6 | 19.9 | 0.000039 |
| | -20 °C, 3 months | Colorless and clear solution | 2.1 | 96.4 | 1.5 | 23.2 | 56.7 | 20.1 | 0.000068 |
| | -20 °C, 6 months | Colorless and clear solution | 2.2 | 96.2 | 1.6 | 24.1 | 55.4 | 20.5 | 0.000099 |
| Note: The appearance was observed after reconstitution of the products. | | | | | | | | | |

Ab1-ADC, Ab2-ADC, Ab3-ADC, and the reference molecule were separately placed in the preferred formula for the Ab1-ADC formulation and subjected to treatment at 2-8 °C or -20 °C for 6 months. No changes in the appearance of the products were observed, but changes were detected in all the molecular size variants (SEC-HPLC), charge variants (CEX-HPLC), and free MMAE content. However, the change rate of related detection indicators decreased as the storage temperature of the products decreased, indicating enhanced stability.

When stored at -20 °C, the SEC-HPLC monomer content of Ab1-ADC, Ab2-ADC, Ab3-ADC, and the reference molecule decreased, mainly accompanied by an increase in the corresponding HMWS content; the CEX-HPLC main peak content decreased, accompanied by an increase in the corresponding acidic peak and basic peak contents; the free MMAE content increased. For Ab1-ADC, Ab2-ADC, and Ab3-ADC, the decrease in the SEC-HPLC monomer content and the increase in the HMWS content were lower than those of the reference molecule; the decrease in the CEX-HPLC main peak content and the increase in the corresponding acidic peak and basic peak contents were lower than those of the reference molecule; the increase in the free MMAE content was lower than that of the reference molecule.

At 2-8 °C, for Ab1-ADC, Ab2-ADC, and Ab3-ADC, the decreases in the SEC-HPLC monomer content and the CEX-HPLC main peak content were significantly lower than that of the reference molecule, the increase in the corresponding HMWS and LMWS contents and the increase in the acidic peak and basic peak contents were significantly lower than those of the reference molecule, and the increase in the free MMAE content was significantly lower than that of the reference molecule. Therefore, the stability of Ab1-ADC, Ab2-ADC, and Ab3-ADC in the formulation formula was significantly superior to that of the reference molecule.

Based on the comprehensive analysis of the evaluation indicators of the products at the two temperatures, the temperatures had an influence on Ab1-ADC, Ab2-ADC, Ab3-ADC, and the reference molecule. Specifically, decreasing the storage temperature enhanced the stability. Under both conditions, Ab1-ADC, Ab2-ADC, and Ab3-ADC were more stable than the reference molecule. Considering the convenience of long-term storage of the products, the four molecules were subjected to lyophilization treatment to evaluate the stability of the products.

**Example 10: Stability of Lyophilized Powder at 2-8 °C and 25 °C for 6 Months**

A preferred formula for the Ab1-ADC formulation was as follows: 10 mg/mL ADC, 20 mmol/L histidine-histidine

hydrochloride, 0.02% polysorbate 20, and 5.5% trehalose (pH 6.0). The ADC molecules obtained by conjugating the Ab1, Ab2 and Ab3 molecules to LND1002 and the reference molecule were buffer-exchanged by ultrafiltration. The samples after buffer exchange were filtered through a 0.22 μm filter membrane. After filtration, the samples were aliquoted into 10 mL cleaned and sterilized injection vials at 30 mg/vial. The four molecules were subjected to lyophilization by using the lyophilization process for Ab1-ADC molecules (specifically, the preparation can be performed with reference to the parameters in Example 6: primary drying: - 25 °C, 0.15 mbar; secondary drying: 30 °C, 0.15 mbar). After the lyophilization was completed, the stability of the four molecules was analyzed at 25 °C and 2-8 °C. Before the stability analysis, about 3.2 mL of water was slowly added to an injection vial using a sterile syringe, and the mixture was reconstituted by gentle swirling until uniform mixing. The evaluation indicators included appearance, molecular size variants (SEC-HPLC), charge variants (CEX-HPLC), and free MMAE content (RP-HPLC/MS). The analysis methods were the same as those in Example 8.

**Table 28: Comparison of stability of lyophilized formulations of four molecules at 25 °C**

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab1-ADC | 0 d | White porous solid | 1.4 | 97.1 | 1.5 | 21.5 | 57.6 | 20.9 | 0.000055 |
| | 25 °C 3 months | White porous solid | 1.6 | 96.8 | 1.6 | 22.3 | 56.2 | 21.5 | 0.00031 |
| | 25 °C 6 months | White porous solid | 2.0 | 96.2 | 1.8 | 23.6 | 54.3 | 22.1 | 0.00056 |
| Ab2-ADC | 0 d | White porous solid | 1.3 | 97.2 | 1.5 | 21.9 | 57.1 | 21.0 | 0.000063 |
| | 25 °C 3 months | White porous solid | 1.6 | 96.7 | 1.7 | 22.8 | 55.4 | 21.8 | 0.00033 |
| | 25 °C 6 months | White porous solid | 1.9 | 96.3 | 1.8 | 24.1 | 53.5 | 22.4 | 0.00058 |
| Ab3-ADC | 0 d | White porous solid | 1.2 | 97.7 | 1.1 | 22.2 | 57.7 | 20.1 | 0.000046 |
| | 25 °C 3 months | White porous solid | 1.4 | 97.3 | 1.3 | 23.2 | 56.0 | 20.8 | 0.00029 |
| | 25 °C 6 months | White porous solid | 1.7 | 96.9 | 1.4 | 24.1 | 54.5 | 21.4 | 0.00052 |
| Reference molecule | 0 d | White porous solid | 1.8 | 96.6 | 1.6 | 23.9 | 56.4 | 19.7 | 0.000078 |
| | 25 °C 3 months | White porous solid | 2.1 | 95.9 | 2.0 | 25.8 | 53.2 | 21.0 | 0.00057 |
| | 25 °C 6 months | White porous solid | 2.6 | 95.1 | 2.3 | 27.9 | 50.1 | 22.0 | 0.0013 |

Ab1-ADC, Ab2-ADC, Ab3-ADC, and the reference molecule were separately placed in the preferred formula for the Ab1-ADC formulation, lyophilized, and then stored at 25 °C for 6 months. No changes in the appearance of the products were observed. Changes were detected in all the molecular size variants (SEC-HPLC), charge variants (CEX-HPLC), and free MMAE content, mainly manifested as a decrease in the SEC-HPLC monomer content, accompanied by an increase in the corresponding HMWS content; a decrease in the CEX-HPLC main peak content, accompanied by an increase in the corresponding acidic peak and basic peak contents; and an increase in the free MMAE content. For the Ab1-ADC, Ab2-ADC, and Ab3-ADC molecules, the decrease in the SEC-HPLC monomer content was lower than that of the reference molecule, the decrease in the CEX-HPLC main peak content and the increase in the corresponding acidic peak and basic peak contents were significantly lower than those of the reference molecule, and the increase in the MMAE content was

significantly lower than that of the reference molecule. The CEX-HPLC indicator for the charge variants and the changes in the free MMAE content of the Ab1-ADC, Ab2-ADC, and Ab3-ADC molecules showed significant differences from those of the reference molecule. After being stored at 25 °C after the same lyophilization process, the stability of the Ab1-ADC, Ab2-ADC, and Ab3-ADC molecules was significantly superior to that of the reference molecule.

**Table 29. Comparison of stability of lyophilized formulations of four molecules at 2-8 °C**

| Sample name | Detection time | Detection indicator | | | | | | | |
|---|---|---|---|---|---|---|---|---|---|
| | | Appearance | SEC-HPLC(%) | | | CEX-HPLC(%) | | | Free MMAE content (%) |
| | | | HWMS | Monomer | LMWS | Acidic peak | Main peak | Basic peak | |
| Ab1-ADC | 0 d | White porous solid | 1.4 | 97.1 | 1.5 | 21.5 | 57.6 | 20.9 | 0.000055 |
| | 2-8 °C, 3 months | White porous solid | 1.5 | 96.9 | 1.6 | 21.8 | 56.9 | 21.3 | 0.000098 |
| | 2-8 °C, 6 months | White porous solid | 1.6 | 96.8 | 1.6 | 22.2 | 56.3 | 21.5 | 0.00015 |
| Ab2-ADC | 0 d | White porous solid | 1.3 | 97.2 | 1.5 | 21.9 | 57.1 | 21.0 | 0.000063 |
| | 2-8 °C, 3 months | White porous solid | 1.5 | 96.9 | 1.6 | 22.2 | 56.7 | 21.1 | 0.00011 |
| | 2-8 °C, 6 months | White porous solid | 1.5 | 96.9 | 1.6 | 22.7 | 55.8 | 21.5 | 0.00017 |
| Ab3-ADC | 0 d | White porous solid | 1.2 | 97.7 | 1.1 | 22.2 | 57.7 | 20.1 | 0.000046 |
| | 2-8 °C, 3 months | White porous solid | 1.3 | 97.6 | 1.1 | 22.5 | 57.1 | 20.4 | 0.0001 |
| | 2-8 °C, 6 months | White porous solid | 1.3 | 97.5 | 1.2 | 22.8 | 56.6 | 20.6 | 0.00014 |
| Reference molecule | 0 d | White porous solid | 1.8 | 96.6 | 1.6 | 23.9 | 56.4 | 19.7 | 0.000078 |
| | 2-8 °C, 3 months | White porous solid | 2.1 | 96.3 | 1.6 | 25.1 | 54.8 | 20.1 | 0.00021 |
| | 2-8 °C, 6 months | White porous solid | 2.3 | 95.8 | 1.9 | 26.0 | 53.4 | 20.6 | 0.00038 |

Ab1-ADC, Ab2-ADC, Ab3-ADC, and the reference molecule were separately placed in the preferred formula for the Ab1-ADC formulation, lyophilized, and then refrigerated at 2-8 °C for 6 months. Compared with the liquid formulation consisting of the same formula under the same storage conditions before lyophilization, the decreases in the SEC-HPLC monomer content and CEX-HPLC main peak content and the increase in the free MMAE content were significantly lower, indicating a significant difference in stability from the liquid formulations. The stability of the lyophilized samples was significantly better than that of the liquid formulations. After lyophilization and storage at 2-8 °C, for the Ab1-ADC, Ab2-ADC and Ab3-ADC molecules, the decrease in the CEX-HPLC main peak content and the increase in the corresponding acidic peak content were lower than those of the reference molecule, and the increase in the MMAE content was lower than that of the reference molecule. Compared with the storage condition of 25 °C, when stored at 2-8 °C, for the four molecules, the increase rate of the free MMAE significantly decreased, the content of the molecular size variant HMWS decreased, and the changes in the charge variant CEX-HPLC main peak, acidic peak and basic peak contents significantly decreased. The refrigeration condition of 2-8 °C is suitable for long-term storage of the samples.

The above descriptions are only preferred embodiments, which are merely used as examples and do not limit the combination of features necessary for implementing the present disclosure. The titles provided are not intended to limit the various embodiments of the present disclosure. Terms such as "comprise", "contain", and "include" are not intended to be limiting. In addition, unless otherwise stated, when a term is not modified by a numerical term, the singular form shall

include the plural form, and "or" means "and/or". Unless otherwise defined herein, all technical and scientific terms used herein have the same meaning as commonly understood by those skilled in the art.

All publications and patents mentioned in the present application are incorporated herein by reference. Various modifications and variations of the methods and compositions described herein will be apparent to those skilled in the art without departing from the scope and spirit of the present disclosure. Although the present disclosure is described by way of specific preferred embodiments, it should be understood that the present disclosure as claimed should not be unduly limited to such specific embodiments. Indeed, various variations of the described modes for implementing the present disclosure which are obvious to those skilled in the related art are intended to be included within the scope of the appended claims.

Table 30: Sequences involved in the present disclosure

| Antibody | Sequence information | |
|---|---|---|
| Antibody CDR The numbering scheme for CDRs is IMGT | Heavy chain<br>CDR1: GYTFTSYY<br>CDR2: IYPGNVNT<br>CDR3: ARGIYYFDY<br>Light chain<br>CDR1: QSVNND<br>CDR2: YAS<br>CDR3: HQDYSSPFT | <br>SEQ ID NO. 1<br>SEQ ID NO. 2<br>SEQ ID NO. 3<br><br>SEQ ID NO. 4<br>SEQ ID NO. 5<br>SEQ ID NO. 6 |
| Humanized antibody SWY2001-Ab1 | SWY2001-Ab1-VH<br>QVQLVQSGAEVKKPGASVKVSCKASGYTFTSYYIHWVRQAPGQRLEWMGWI<br>YPGNVNTKYNENFRDRVTITRDTSASTAYMELSSLRSEDTAVYYCARGIYYFD<br>YWGQGTLVTVSS<br>SEQ ID NO.7<br>SWY2001-Ab1-VL<br>IIQMTQSPKFLSASVGDRVTITCKASQSVNNDVAWYQQKPGQSPKLLIYYASN<br>RDTGVPDRFSGSGSGTDFTLTISSLQPEDFATYFCHQDYSSPFTFGGGTKVEIK<br>SEQ ID NO.8 | |
| Transglutaminase | DSDERVTPPAEPLDRMPDPYRPSYGRAETIVNNYIRKWQQVYSHRDGRKQQM<br>TEEQREWLSYGCVGVTWVNSGQYPTNRLAFAFFDEDKYKNELKNGRPRSGE<br>TRAEFEGRVAKDSFDEAKGFQRARDVASVMNKALENAHDEGAYLDNLKKEL<br>ANGNDALRNEDARSPFYSALRNTPSFKDRNGGNHDPSKMKAVIYSKHFWSG<br>QDRSGSSDKRKYGDPEAFRPDRGTGLVDMSRDRNIPRSPTSPGESFVNFDYGW<br>FGAQTEADADKTVWTHGNHYHAPNGSLGAMHVYESKFRNWSDGYSDFDRG<br>AYVVTFVPKSWNTAPDKVTQGWP<br>SEQ ID NO.14 | |

(continued)

| Antibody | Sequence information |
|---|---|
| Humanized anti-body SWY2001-Ab2 | SWY2001-Ab2-VH<br>QVQLVQSGAEVKKPGASVKVSCKASGYTFTSYYIHWVRQAPGQRLEWMGWI YPGNVNTKYNENFRDRVTITRDTSASTAYMELSSLRSEDTAVYYCARGIYYFD YWGQGTLVTVSS<br>SEQ ID NO.7<br>SWY2001-Ab2-VC<br>AIQMTQSPSSLSASVGDRVTITCKASQSVNNDVAWYQQKPGKAPKLLIYYAS NRDTGVPSRFSGSGSGTDFTLTISSLQAEDLAVYFCHQDYSSPFTFGGGTKVEI K<br>SEQ ID NO.9 |
| Humanized anti-body SWY2001-Ab3 | SWY2001-Ab3-VH<br>QVQLVQSGAEVKKPGASVKVSCKASGYTFTSYYIHWVRQAPGQRLEWMGWI YPGNVNTKYNENFRDRVTITRDTSASTAYMELSSLRSEDTAVYYCARGIYYFD YWGQGTLVTVSS<br>SEQ ID NO.7<br>SWY2001-Ab3-VC<br>AIQMTQSPSSLSASVGDRVTITCKASQSVNNDVAWYQQKPGKAPKLLIYYAS NRDTGVPSRFSGSGSGTDFTLTISSLQPEDFATYFCHQDYSSPFTFGGGTKVEIK<br>SEQ ID NO.10 |

(continued)

| Antibody | Sequence information |
|---|---|
| Constant region | HC1<br>ASTKGPSVFPLAPSSKSTSGGTAALGCLVKDYFPEPVTVSWNSGALTSGVHTF<br>PAVLQSSGLYSLSSVVTVPSSSLGTQTYICNVNHKPSNTKVDKRVEPKSCDKT<br>HTCPPCPAPELLGGPSVFLFPPKPKDTLMISRTPEVTCVVVDVSHEDPEVKFNW<br>YVDGVEVHNAKTKPREEQYNSTYRVVSVLTVLHQDWLNGKEYKCKVSNKA<br>LPAPIEKTISKAKGQPREPQVYTLPPSREEMTKNQVSLTCLVKGFYPSDIAVEW<br>ESNGQPENNYKTTPPVLDSDGSFFLYSKLTVDKSRWQQGNVFSCSVMHEALH<br>NHYTQKSLSLSPGK(SEQ ID NO.11)<br>LC<br>RTVAAPSVFIFPPSDEQLKSGTASVVCLLNNFYPREAKVQWKVDNALQSGNS<br>QESVTEQDSKDSTYSLSSTLTLSKADYEKHKVYACEVTHQGLSSPVTKSFNRG<br>EC(SEQ ID NO.13)<br>HC2<br>ASTKGPSVFPLAPSSKSTSGGTAALGCLVKDYFPEPVTVSWNSGALTSGVHTF<br>PAVLQSSGLYSLSSVVTVPSSSLGTQTYICNVNHKPSNTKVDKKVEPKSCDKT<br>HTCPPCPAPELLGGPSVFLFPPKPKDTLMISRTPEVTCVVVDVSHEDPEVKFNW<br>YVDGVEVHNAKTKPREEQYNSTYRVVSVLTVLHQDWLNGKEYKCKVSNKA<br>LPAPIEKTISKAKGQPREPQVYTLPPSREEMTKNQVSLTCLVKGFYPSDIAVEW<br>ESNGQPENNYKTTPPVLDSDGSFFLYSKLTVDKSRWQQGNVFSCSVMHEALH<br>NHYTQKSLSLSPGK(SEQ ID NO.12) |

**Claims**

1. A pharmaceutical composition comprising a Nectin-4 antibody-drug conjugate, comprising: (i) an antibody-drug conjugate, (ii) a buffer, (iii) a stabilizer, and (iv) a surfactant, wherein the antibody-drug conjugate is formed by conjugating a drug-linker represented by formula I to an anti-Nectin-4 monoclonal antibody;

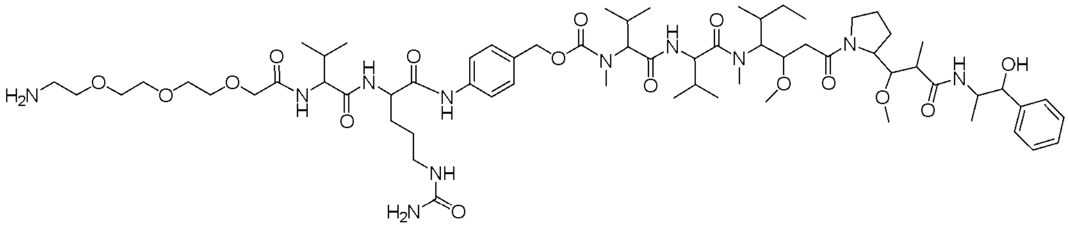

formula I

the antibody comprises a heavy chain and a light chain, wherein the heavy chain comprises three CDR regions having amino acid sequences set forth in SEQ ID NOs: 1, 2 and 3, respectively, and/or the light chain comprises three CDR regions having amino acid sequences set forth in SEQ ID NOs: 4, 5 and 6, respectively.

2. The pharmaceutical composition according to claim 1, having one or more of the following characteristics: (a) the surfactant is polysorbate 20 or polysorbate 80, preferably polysorbate 20; (b) the stabilizer is sucrose or trehalose, preferably trehalose; (c) the buffer is histidine-histidine hydrochloride, succinic acid-sodium succinate, or citric acid-sodium citrate, preferably histidine-histidine hydrochloride.

3. The pharmaceutical composition according to any one of claims 1-2, comprising: histidine-histidine hydrochloride: 5-30 mM, polysorbate 20 or polysorbate 80: 0.02-0.08 wt%, trehalose: 4-8 wt%, and the antibody-drug conjugate: 5-50 mg/mL, pH 5.0-6.5.

4. The pharmaceutical composition according to any one of claims 1-3, comprising: histidine-histidine hydrochloride: 10-30 mM, polysorbate 20 or polysorbate 80: 0.02-0.04 wt%, trehalose: 5-7 wt%, and the antibody-drug conjugate: 10-20 mg/mL, pH 5.0-6.0.

5. The pharmaceutical composition according to any one of claims 1-4, comprising histidine-histidine hydrochloride: 20 mM, polysorbate 20: 0.02 wt%, trehalose: 5.5 wt%, and the antibody-drug conjugate: 10 mg/mL, pH 6.0.

6. The pharmaceutical composition according to any one of claims 1-5, further comprising water.

7. The pharmaceutical composition according to any one of claims 1-6, wherein the pharmaceutical composition can be a liquid formulation, a lyophilized formulation, or a powder for injection, preferably a liquid formulation or a lyophilized formulation.

8. Use of the pharmaceutical composition according to any one of claims 1-7 for manufacturing a medicament for the treatment of cancer.

9. The use according to claim 8, wherein the cancer is a Nectin-4 positive cancer, including but not limited to: bladder cancer, brain cancer, breast cancer, cervical cancer, thoracic tumors, endometrial cancer, esophageal squamous carcinoma, gastric cancer, head tumors, pancreatic cancer, cholangiocarcinoma, colorectal cancer, eye cancer, head and neck squamous cell carcinoma, urothelial carcinoma, kidney cancer, liver cancer, lymph node cancer, lung cancer, oral cancer, neck tumors, ovarian cancer, prostate cancer, testicular cancer, laryngeal cancer, uterine cancer, melanoma, salivary gland cancer, fibrosarcoma, soft tissue sarcoma, and osteosarcoma.

10. The pharmaceutical composition according to any one of claims 1-9, wherein the heavy chain of the antibody comprises a heavy chain variable region having an amino acid sequence set forth in SEQ ID NO: 7, and/or the light chain of the antibody comprises a light chain variable region selected from an amino acid sequence set forth in any one of SEQ ID NOs: 8-10.

**11.** The pharmaceutical composition according to any one of claims 1-10, wherein the heavy chain of the antibody comprises a heavy chain constant region having an amino acid sequence set forth in SEQ ID NO: 11 or 12, and/or the light chain of the antibody comprises a light chain constant region selected from an amino acid sequence set forth in SEQ ID NO: 13.

**12.** A method for preparing the pharmaceutical composition according to any one of claims 1-11, comprising the following steps: preparing an anti-Nectin-4 antibody-drug conjugate, buffer-exchanging the antibody-drug conjugate into other components of a liquid pharmaceutical composition, performing sterilizing filtration, and performing aseptic filling.

**13.** A method for preparing a lyophilized formulation of the pharmaceutical composition according to any one of claims 1-11, wherein the method comprises the following steps: pre-freezing the pharmaceutical composition according to any one of claims 1-10, vacuumizing, performing primary drying, and performing secondary drying.

**14.** The method according to claim 13, wherein the primary drying is performed under a condition of about -25 °C, 0.15 mbar.

**15.** The method according to claim 13, wherein the secondary drying is performed at a temperature of about 30 °C.

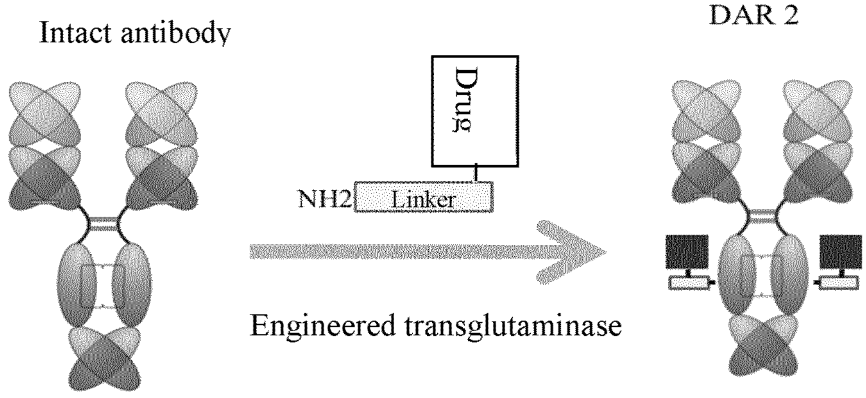
Intact antibody
DAR 2
Drug
NH2
Linker
Engineered transglutaminase

FIG. 1

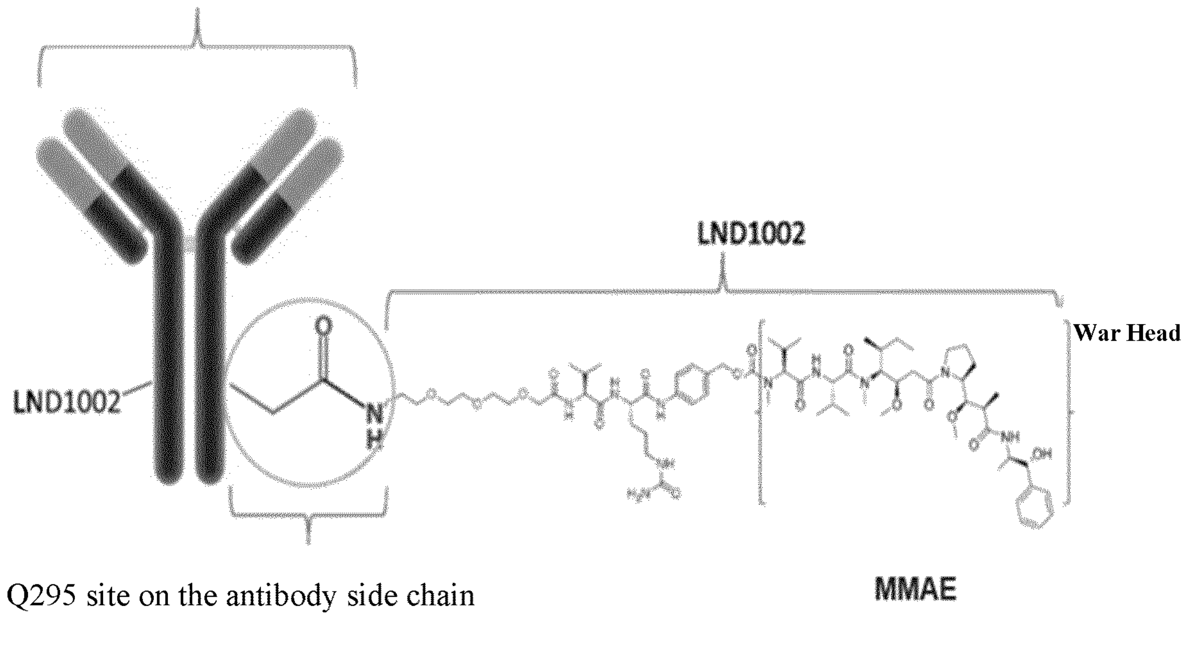
LND1002
LND1002
War Head
Q295 site on the antibody side chain
MMAE

FIG. 2

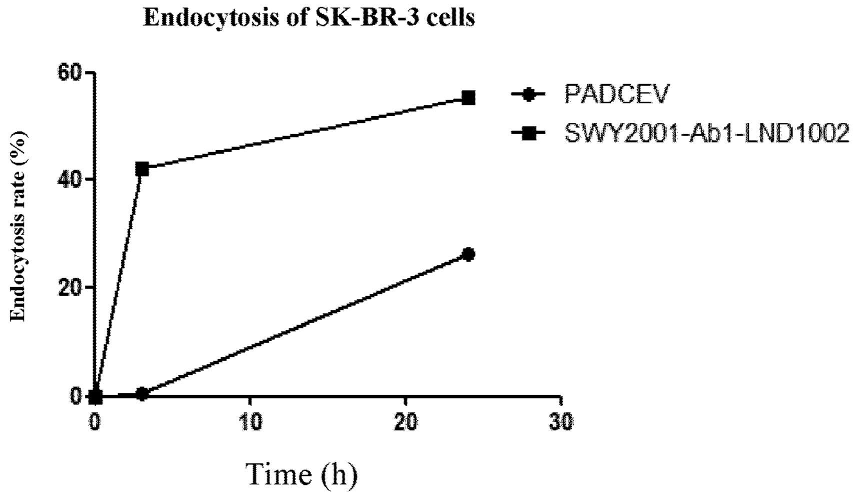
Endocytosis of SK-BR-3 cells
Endocytosis rate (%)
60
40
20
0
0
10
20
30
Time (h)
PADCEV
SWY2001-Ab1-LND1002

FIG. 3

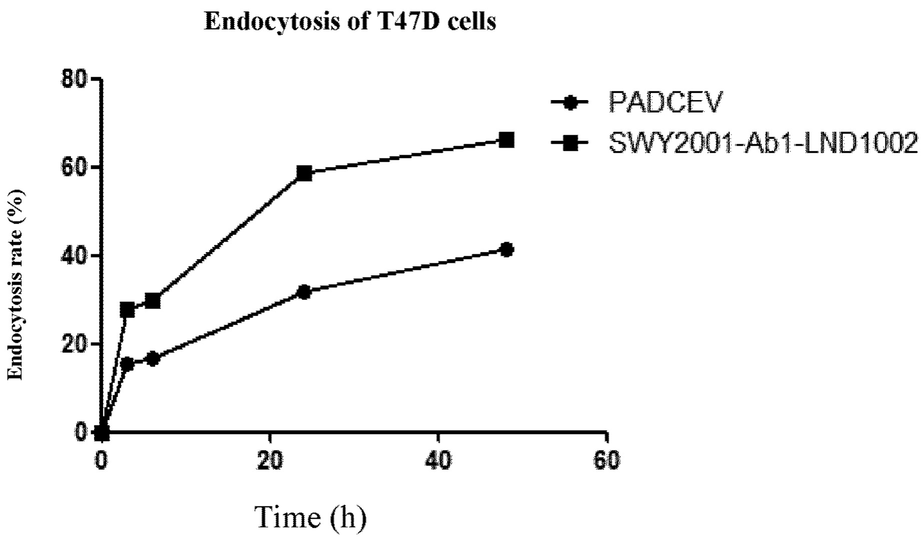
Endocytosis of T47D cells
Endocytosis rate (%)
80
60
40
20
0
0
20
40
60
Time (h)
PADCEV
SWY2001-Ab1-LND1002

FIG. 4

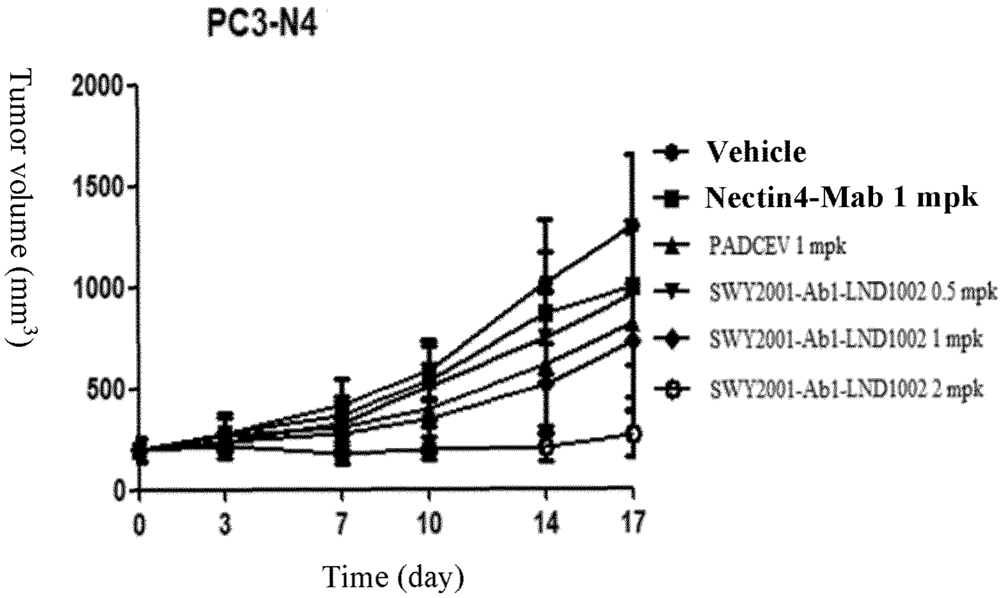
PC3-N4
Tumor volume (mm³)
2000
1500
1000
500
0
0
3
7
10
14
17
Time (day)
Vehicle
Nectin4-Mab 1 mpk
PADCEV 1 mpk
SWY2001-Ab1-LND1002 0.5 mpk
SWY2001-Ab1-LND1002 1 mpk
SWY2001-Ab1-LND1002 2 mpk

FIG. 5

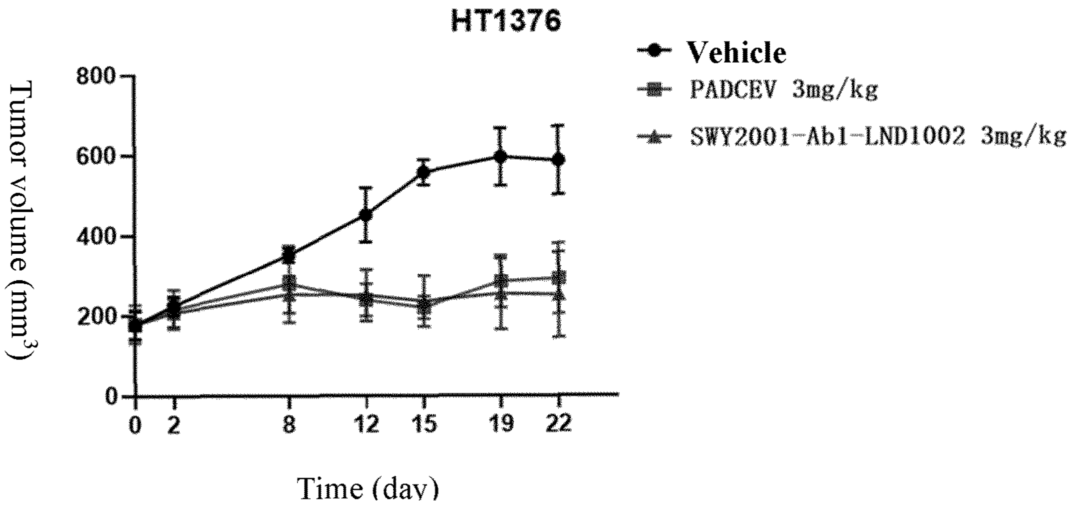
HT1376
Tumor volume (mm³)
800
600
400
200
0
0
2
8
12
15
19
22
Time (day)
Vehicle
PADCEV 3mg/kg
SWY2001-Ab1-LND1002 3mg/kg

FIG. 6

**INTERNATIONAL SEARCH REPORT**

International application No.
**PCT/CN2024/078768**

**A. CLASSIFICATION OF SUBJECT MATTER**

A61K 47/68(2017.01)i; A61K39/395(2006.01)i; C07K16/30(2006.01)i; A61P35/00(2006.01)i; C07K16/28(2006.01)i; C12N15/13(2006.01)i; A61K31/704(2006.01)i

According to International Patent Classification (IPC) or to both national classification and IPC

**B. FIELDS SEARCHED**

Minimum documentation searched (classification system followed by classification symbols)

IPC: A61K,C07K,C12N,A61P

Documentation searched other than minimum documentation to the extent that such documents are included in the fields searched

Electronic data base consulted during the international search (name of data base and, where practicable, search terms used)

CNABS, CNTXT, SIPOABS, DWPIWOTXT, USTXT, EPTXT, CNKI, Pubmed, NCBI, EMBL, STN, 中国专利生物序列检索系统, China Patents Biological Sequence Search System, ISI Web of Science: 解茹, 韩卫强, 王旭明, 苏利, 曹卫荣, Nectin-4, 抗体, PVRL4, antibody, CDR, 可变区, 重链, 轻链, 石药集团, 巨石生物, CSPC MEGALITH, 缀合物, 偶联物, 连接子, 接头, 偶联, 癌, 肿瘤, cancer, tumor, ADC, SEQ ID NOs: 1-8, SEQ ID NOs: 11-13, STN structural formula search, conjugate, MMAE, Monomethyl auristatin E, 甲基澳瑞他汀, SWY2001, Val-Cit

**C. DOCUMENTS CONSIDERED TO BE RELEVANT**

| Category* | Citation of document, with indication, where appropriate, of the relevant passages | Relevant to claim No. |
|---|---|---|
| PX | WO 2023025243 A1 (CSPC JUSHI BIOLOGICAL PHARMACEUTICAL CO., LTD.) 02 March 2023 (2023-03-02)<br>description, page 5, lines 4-15, page 11, line 7, and pages 16-17, table 2, embodiments 5-9 | 1-15 |
| A | CN 113527486 A (MABWELL (SHANGHAI) BIOSCIENCE CO., LTD.) 22 October 2021 (2021-10-22)<br>claims 1-14, and description, embodiments 7-8, paragraphs [0008]-[0027] | 1-15 |
| A | US 2018243434 A1 (INSERM (INSTITUT NAT DE LA SANTÉ ET DE LA RECH MÉDICALE)) 30 August 2018 (2018-08-30)<br>description, embodiment 1 | 1-15 |
| A | CN 114901308 A (CSPC JUSHI BIOLOGICAL PHARMACEUTICAL CO., LTD.) 12 August 2022 (2022-08-12)<br>claims 18-20 | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2024** | **16 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/078768**

**C. DOCUMENTS CONSIDERED TO BE RELEVANT**

| Category* | Citation of document, with indication, where appropriate, of the relevant passages | Relevant to claim No. |
|---|---|---|
| A | CN 112189020 A (SEATTLE GENETICS INC.) 05 January 2021 (2021-01-05)<br>description, embodiments 1-3 | 1-15 |
| A | 周玥 等 (ZHOU, Yue et al.). "抗体偶联药物研究进展 (Research and Development of Antibody-Drug Conjugates)"<br>*中国海洋药物 (Chinese Journal of Marine Drugs),*<br>Vol. 35, No. 05, 15 October 2016 (2016-10-15),<br>pages 89-94 | 1-15 |

Form PCT/ISA/210 (second sheet) (July 2022)

**INTERNATIONAL SEARCH REPORT**

International application No.
**PCT/CN2024/078768**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/078768**

| Patent document cited in search report | | | Publication date (day/month/year) | Patent family member(s) | | | Publication date (day/month/year) |
|---|---|---|---|---|---|---|---|
| WO | 2023025243 | A1 | 02 March 2023 | CA | 3230122 | A1 | 02 March 2023 |
| | | | | AU | 2022335573 | A1 | 07 March 2024 |
| | | | | CN | 117897407 | A | 16 April 2024 |
| CN | 113527486 | A | 22 October 2021 | None | | | |
| US | 2018243434 | A1 | 30 August 2018 | JP | 2018531913 | A | 01 November 2018 |
| | | | | JP | 6985252 | B2 | 22 December 2021 |
| | | | | US | 10675357 | B2 | 09 June 2020 |
| | | | | WO | 2017042210 | A1 | 16 March 2017 |
| | | | | EP | 3347048 | A1 | 18 July 2018 |
| | | | | EP | 3347048 | B1 | 01 April 2020 |
| | | | | ES | 2794557 | T3 | 18 November 2020 |
| CN | 114901308 | A | 12 August 2022 | WO | 2021086981 | A1 | 06 May 2021 |
| CN | 112189020 | A | 05 January 2021 | US | 2019290775 | A1 | 26 September 2019 |
| | | | | SG | 11202009264 | WA | 29 October 2020 |
| | | | | MA | 52135 | A | 27 January 2021 |
| | | | | AU | 2019240403 | A1 | 08 October 2020 |
| | | | | TW | 202003047 | A | 16 January 2020 |
| | | | | KR | 20200135841 | A | 03 December 2020 |
| | | | | BR | 112020018948 | A2 | 05 January 2021 |
| | | | | IL | 277338 | A | 29 October 2020 |
| | | | | CA | 3093731 | A1 | 26 September 2019 |
| | | | | JP | 2021518397 | A | 02 August 2021 |
| | | | | MX | 2020009842 | A | 15 October 2020 |
| | | | | EP | 3768714 | A1 | 27 January 2021 |
| | | | | WO | 2019183438 | A1 | 26 September 2019 |
| | | | | US | 2023110128 | A1 | 13 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- High-Performance Liquid Chromatography. Chinese Pharmacopoeia. 2020, vol. IV
- Determination of Size Variants of Monoclonal Antibodies. Chinese Pharmacopoeia. 2020, vol. IV
- Monoclonal Antibody Charge Variant Determination Method. Chinese Pharmacopoeia. 2020, vol. IV
- Mass Spectrometry. Chinese Pharmacopoeia. 2020, vol. IV